# EUROPÄISCHE PATENTSCHRIFT

(11) **EP 1 080 192 B1**
(45) Veröffentlichungstag und Bekanntmachung des Hinweises auf die Patenterteilung: **28.07.2004**
(21) Anmeldenummer: 99926337.9
(22) Anmeldetag: 20.05.1999
(51) Int. Cl.: C12N 15/11, A61K 31/70, C07H 21/00

(54) **ANTISENSE-OLIGONUKLEOTIDE ZUR BEHANDLUNG VON PROLIFERIERENDEN ZELLEN**
ANTISENSE OLIGONUCLEOTIDES FOR TREATING PROLIFERATING CELLS
OLIGONUCLEOTIDES ANTISENS POUR LE TRAITEMENT DE CELLULES PROLIFERANTES

(30) Priorität: 22.05.1998 DE 19822954
(43) Veröffentlichungstag der Anmeldung: 07.03.2001
(73) Patentinhaber: Faustus Forschungs Cie. Translational Cancer Research GmbH, 04109 Leipzig (DE)
(72) Erfinder: FLAD, Hans-Dieter, D-23845 Borstel (DE); GERDES, Johannes, D-23858 Feldhorst (DE); BÖHLE, Andreas, D-23627 Gross Grönau (DE); Ährchen-Deinert, Irina, D-22964 Steinberg (DE)
(74) Vertreter: Grünecker, Kinkeldey, Stockmair & Schwanhäusser Anwaltssozietät
(86) Internationale Anmeldenummer: PCT/EP1999/003451
(87) Internationale Veröffentlichungsnummer: WO 1999/061607

(56) Entgegenhaltungen:
- SCHLUETER, C. ET AL: "The cell proliferation-associated antigen of antibody Ki-67: A very large ubiquitous nuclear protein with numerous repeated elements, representing new kind of cell cycle-maintaining proteins" J. CELL BIOL. (1993), 123(3), 513-22 , XP000867381 in der Anmeldung erwähnt
- MAESHIMA, YOHEI ET AL: "Antisense oligonucleotides to proliferating cell nuclear antigen and Ki-6 inhibit human mesangial cell proliferation" J. AM. SOC. NEPHROL. 7(10), 2219-2229 , Oktober 1996 (1996-10), XP000867392
- UHLMANN E ET AL: "ANTISENSE OLIGONUCLEOTIDES: A NEW THERAPEUTIC PRINCIPLE" CHEMICAL REVIEWS, Bd. 90, Nr. 4, 1990, Seite 543-584 XP000141412 ISSN: 0009-2665 in der Anmeldung erwähnt

## Beschreibung

Die Erfindung betrifft Oligoribo- und Oligodesoxyribonukleotide, die sich zur Therapie von Krankheitszuständen eignen, die mit einer erhöhten Zellproliferation einhergehen.

Als Antisense-Oligonukleotide werden Nukleinsäurefragmente bezeichnet, deren Sequenz komplementär zum kodierenden oder "Sense"-Strang der DNA oder einer Messenger-RNA (mRNA) ist und die somit in der Lage sind, spezifisch an diese komplementären Zielsequenzen zu binden (hybridisieren). Hierdurch ist eine selektive Beeinflussung zellulärer Prozesse möglich. Antisense-Oligonukleotide haben als Werkzeuge in der Forschung sowie als potentielle Mittel zur antiviralen und Tumortherapie Interesse gefunden (E. Uhlmann, A. Peyman, Chemical Reviews, 90 (1990) 544-584; S. Agrawal, TIBTECH 10 (1992) 152-158) und haben zum Teil bereits das Stadium der klinischen Forschung erreicht (M.D. Matteucci, R.W. Wagner, Nature 384 (196) 20-22).

Ki-67 ist ein zelluläres Protein, das in allen aktiven Phasen des Zellzyklus (G₁, S, G₂ und Mitose) nicht aber während der Ruhephase (G₀) produziert wird. Die Ruhe- oder G₀-Phase beschreibt den Zustand, in dem die Teilungsaktivität der Zelle ruht, d.h die Zellen die aktiven Phasen des Zellzyklusses verlassen haben und sich nicht teilen. Ki-67 ist ein humanes, nukleäres Protein, dessen Expression streng mit der Zellproliferation assoziiert ist. Spezifische Antikörper gegen das Ki-67 Protein werden in der Histopathologie zur Bestimmung des Anteils wachsender Zellen in humanen Tumoren verwendet (J. Gerdes, Seminars in Cancer Biology 1 (1990) 199-206).

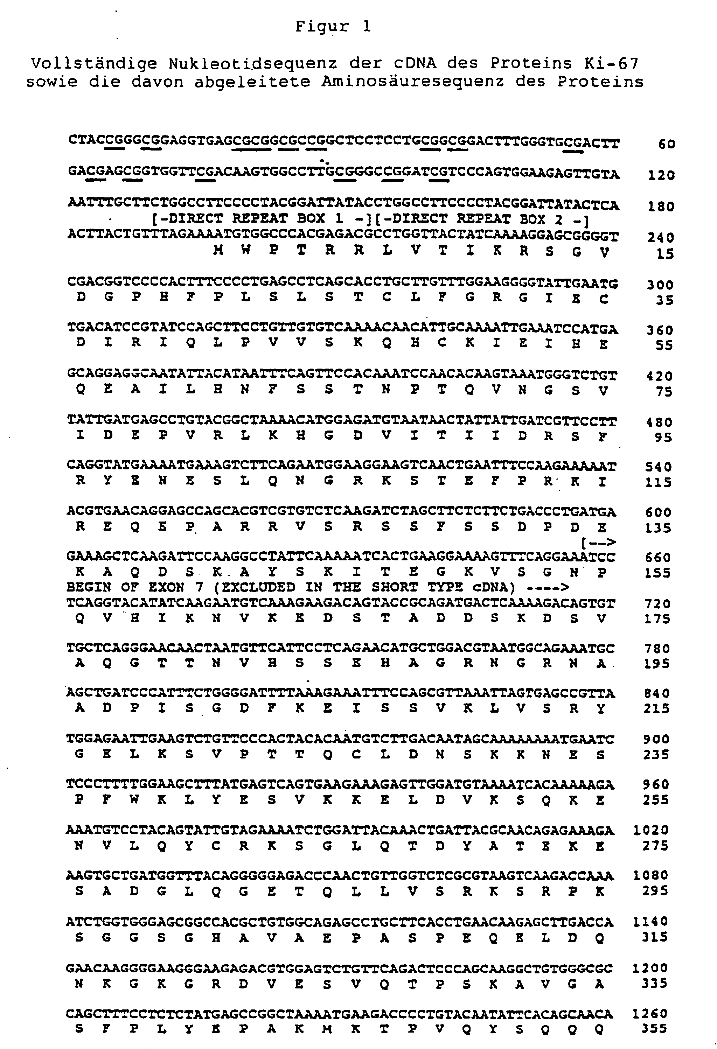

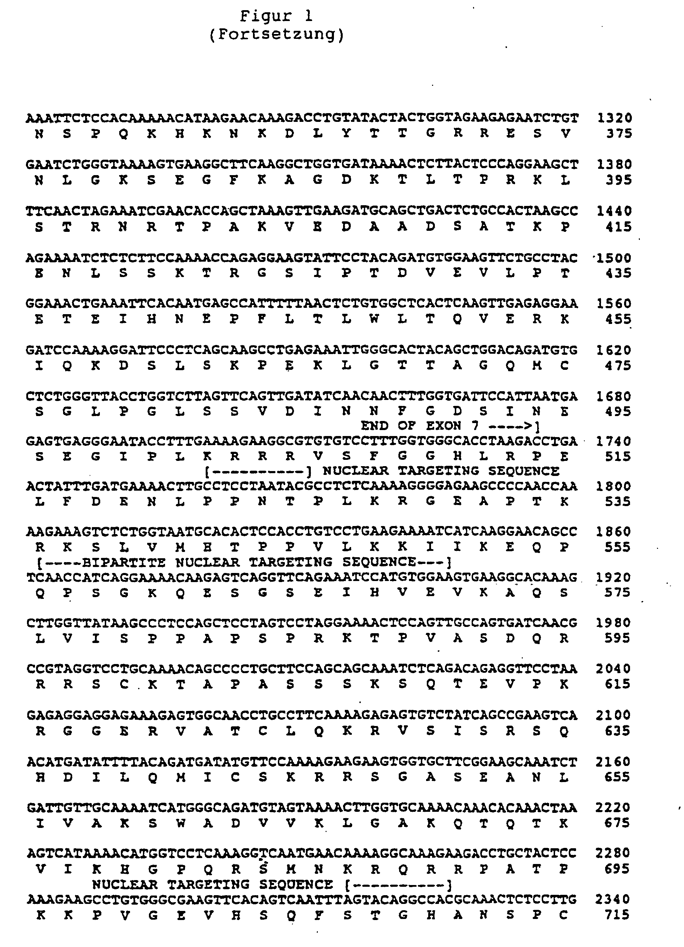

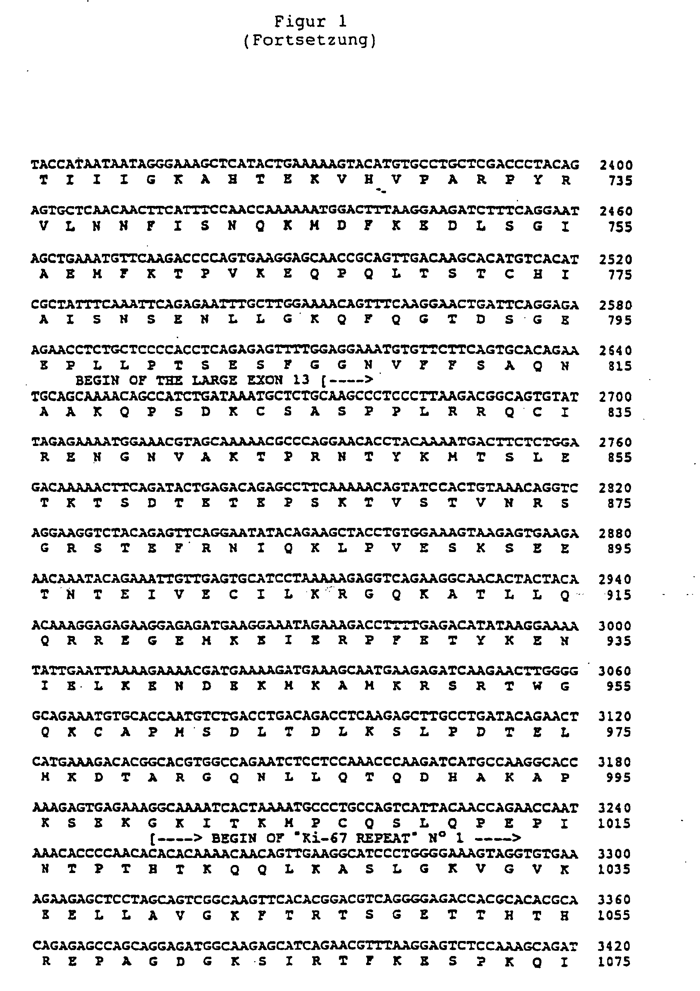

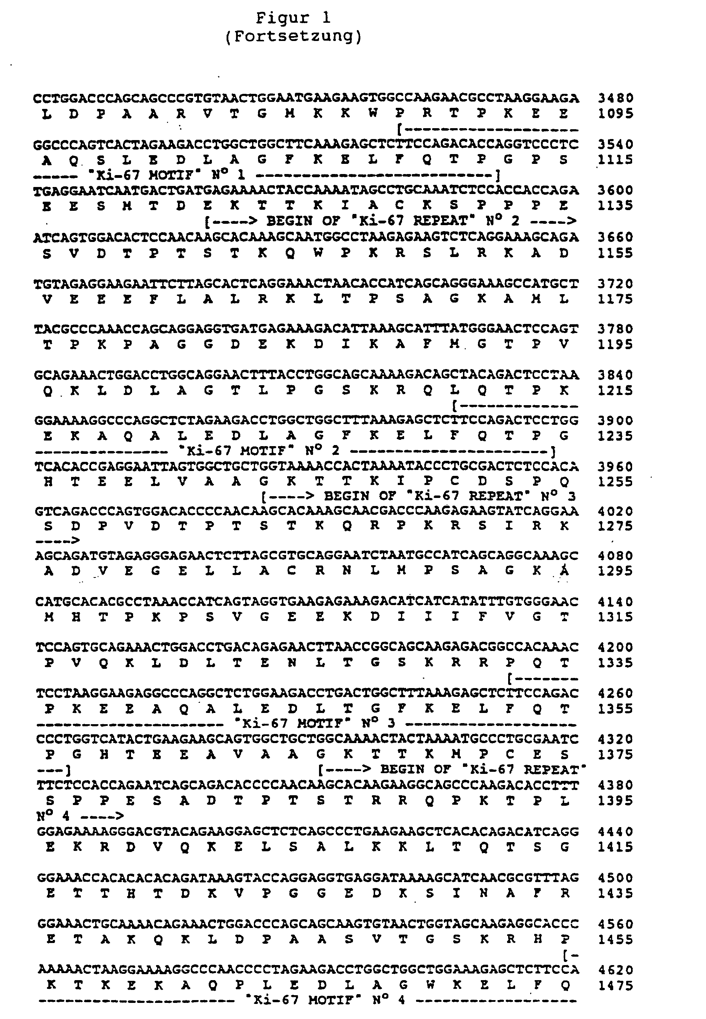

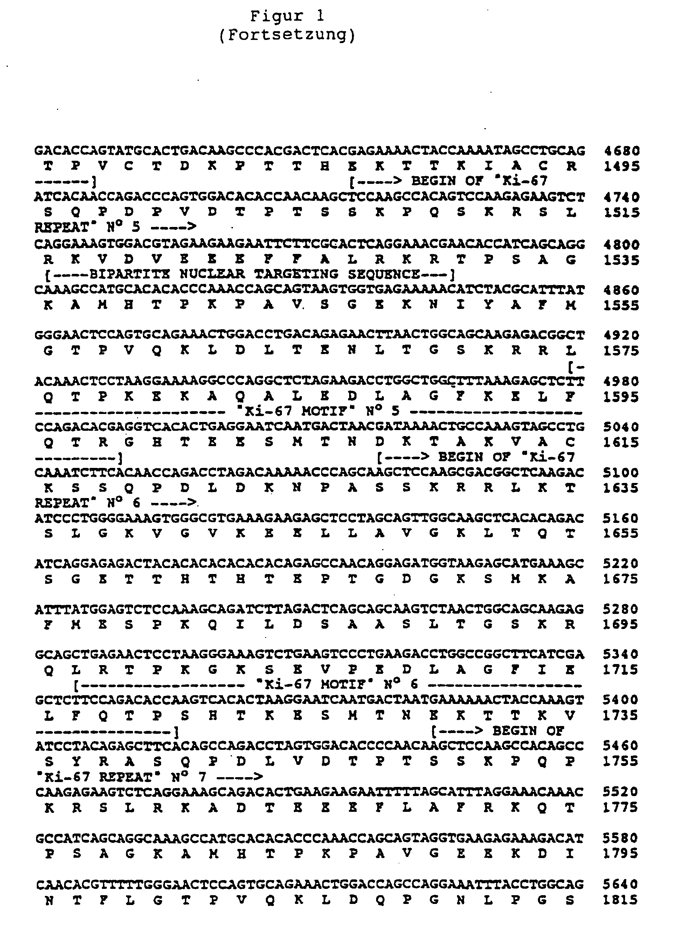

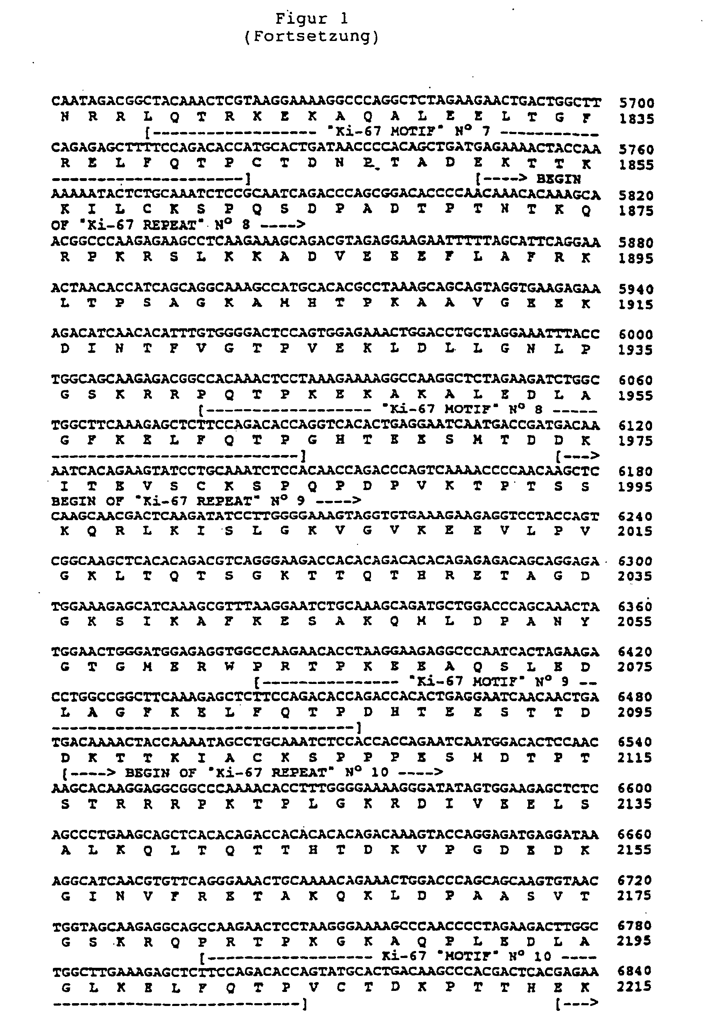

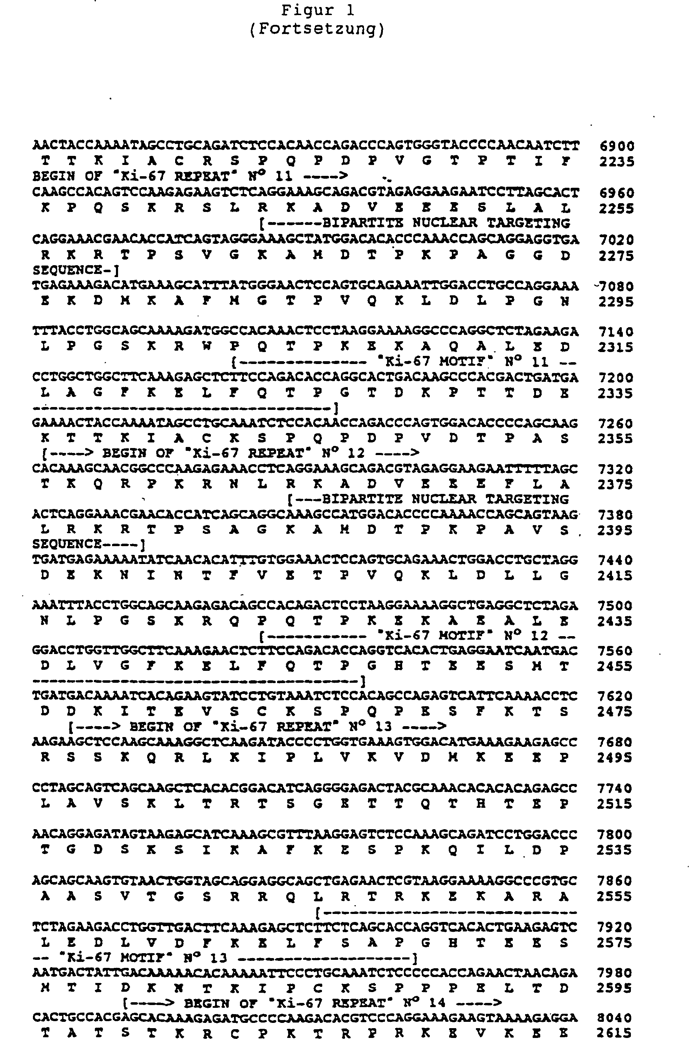

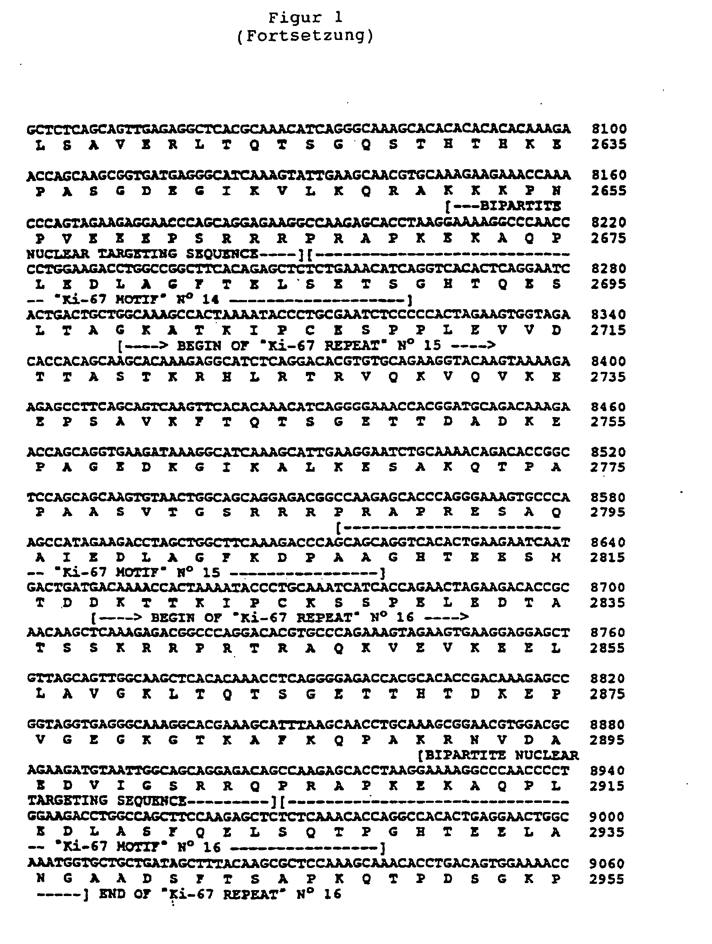

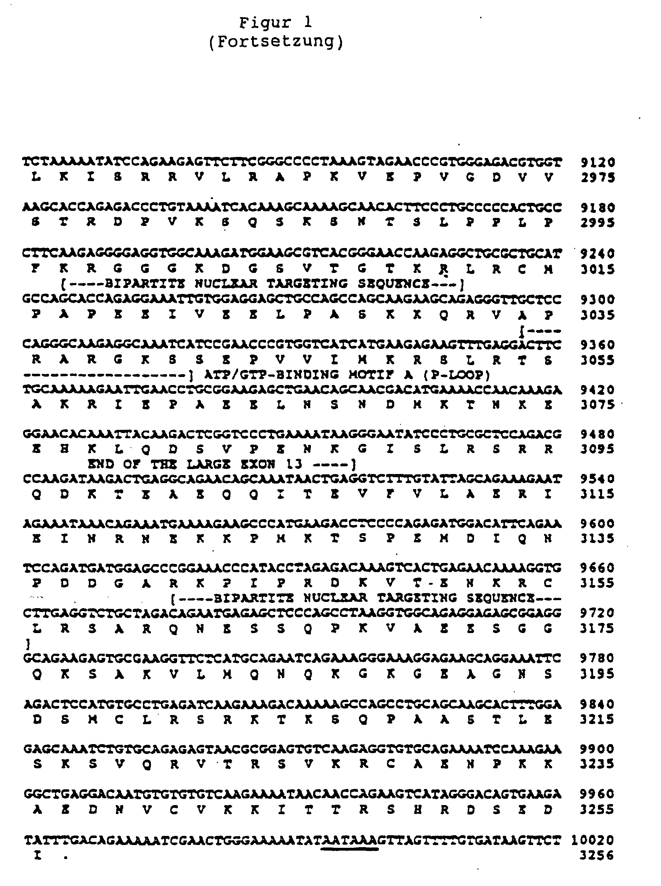

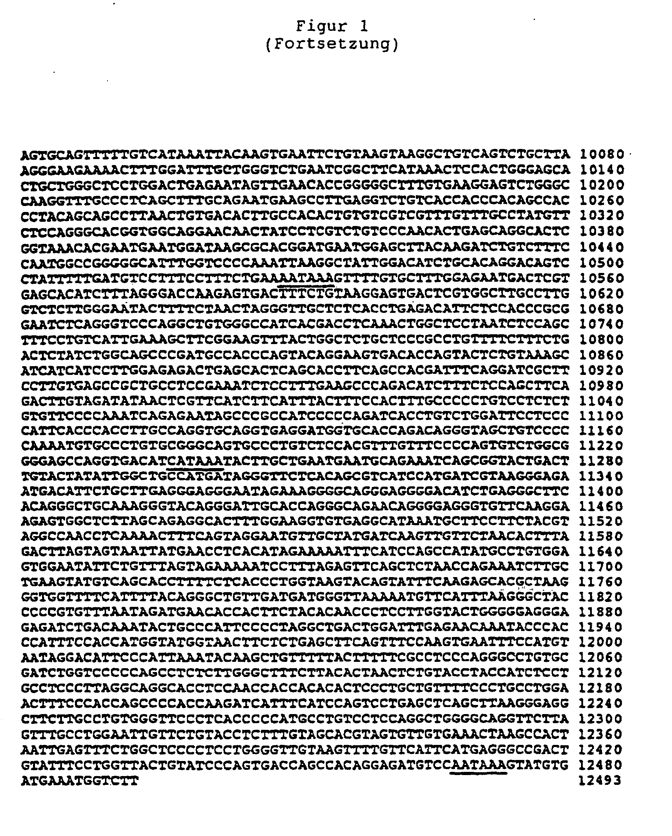

Es wurde zudem gezeigt, daß durch ein 21 Basen umfassendes Ki-67 Protein Antisense-2'-Desoxyoligonukleotid die Proliferation von humanen IM-9-Zellen konzentrationsabhängig inhibiert werden kann (C. Schlüter et al., The Journal of Cell Biology, 123 (1993) 513-522). Die vollständige Nukleotidsequenz der cDNA des Ki-67-Proteins sowie die abgeleitete Aminosäuresequenz sind bekannt (Schlüter et al., a.a.O.). Figur 1 (SEQ ID NO 1) zeigt den sense-Strang der Ki-67 cDNA.

Aufgabe der vorliegenden Erfindung ist die Bereitstellung von Antisense-Oligonukleotiden, die sich zur Therapie von Krankheitszuständen eignen, die mit einer erhöhten Zellproliferation einhergehen. Beispiele solcher Krankheitszustände sind Tumoren, Allergien, Autoimmunerkrankungen, Narbenbildung, Entzündungen und rheumatische Erkrankungen sowie die Unterdrückung von Abwehrreaktionen bei Transplantationen.

Diese Aufgabe wurde durch Oligoribo- oder Oligodesoxyribonukleotide sowie deren physiologisch verträgliche Salze gelöst, die in der Lage sind, mit der für das Protein Ki-67 kodierenden mRNA zu hybridisieren.

Es wurde gefunden, daß die erfindungsgemäßen Oligoribo- oder Oligodesoxyribonukleotide eine zytotoxische und nicht nur inhibierende Wirkung auf proliferierende Zellen wie beispielsweise Tumorzellen haben und den Tod der Zellen bewirken. Dieser Befund ist insofern überraschend, als das Ki-67-Protein in nicht-proliferierenden Zellen nicht nachweisbar und somit für das Überleben der Zellen offensichtlich nicht erforderlich ist.

Bevorzugt sind Oligonukleotide, die bei 37 °C und bei physiologischer Salzkonzentration mit Ki-67 mRNA hybridisieren.

Besonders bevorzugt sind Oligoribo- und Oligodesoxyribonukleotide und insbesondere Oligodesoxyribonukleotide, deren Sequenz zu der in Figur 1 (SEQ ID NO: 1) gezeigten Nukleotidsequenz des sense-Stranges der cDNA von Ki-67 komplementär ist, d.h. bei einer Kettenlänge von 10 Basen maximal 0 bis 4, vorzugsweise 0 bis 2 und ganz besonders bevorzugt keine Fehlpaarungen (mismatches) aufweist.

Als besonders wirksam erwiesen sich weiterhin Oligoribo- und Oligodesoxyribonukleotide, die mit einer Nukleotidsequenz aus dem 5'-Bereich der Ki-67 mRNA hybridisieren, d.h. Oligoribooder Oligodesoxyribonukleotide, die zu dem 5'-Bereich der in Figur 1 gezeigten Sequenz, vorzugsweise zu einem Abschnitt aus dem Bereich von Position 197 bis 2673 oder 2673 bis 9962, besonders bevorzugt 197 bis 220 komplementär sind.

Die erfindungsgemäßen Oligonukleotide weisen vorzugsweise eine Kettenlänge von 12 bis 66 Nukleotiden, besonders bevorzugt 17 bis 46 und ganz besonders bevorzugt 22 bis 46 Nukleotiden auf.

Ganz besonders bevorzugt ist die Sequenz (SEQ ID NO: 3):

Unmodifizierte Oligonukleotide und insbesondere unmodifizierte Oligoribonukleotide sind in hohem Maße dem nukleolytischem Abbau unterworfen und weisen daher nur eine geringe Stabilität und biologische Halbwertszeit auf. Zur Verbesserung der Membrangängigkeit und zur Erhöhung der biologischen Halbwertszeit sind die Basen, Zucker- und/oder Phosphatreste der erfindungsgemäßen Oligonukleotide vorzugsweise modifiziert.

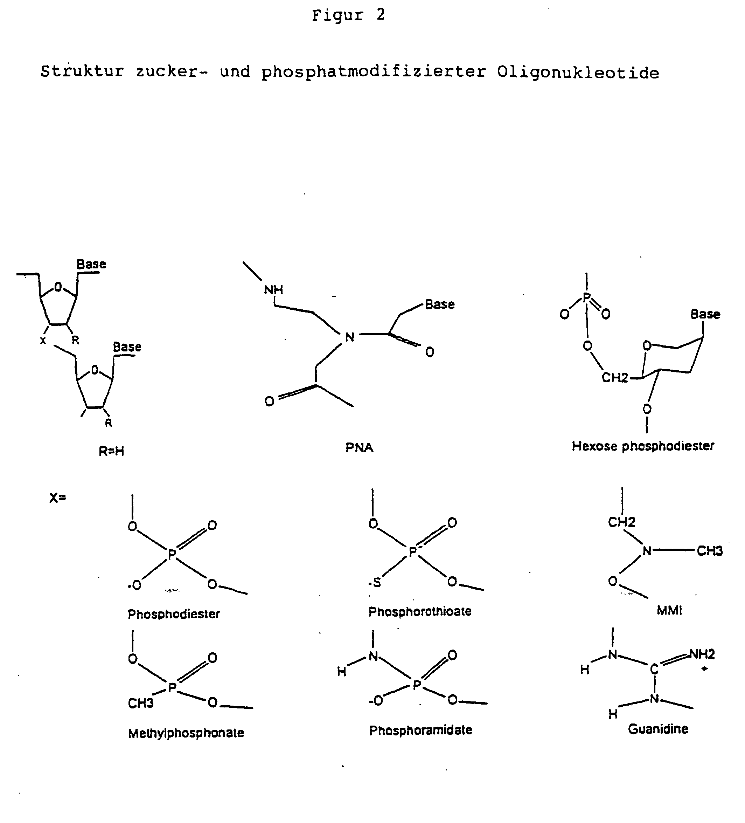

Bevorzugt sind Oligonukleotide, bei denen eine oder mehrere Phosphatgruppen durch Phosphothioat-, Methylphosphonat-, Phosphoramidat-, Methylen(methylimino)- (MMI) und/oder Guanidingruppen ausgetauscht sind. Die Struktur dieser Gruppen ist in Figur 2 gezeigt. Besonders bevorzugt sind thiolierte Oligonukeotide, d.h. Oligonukleotide bei denen Phosphatgruppen durch Phospho-thioatgruppen ausgetauscht sind. Es können eine oder mehrere der Phosphatgruppen des Oligonukleotids modifiziert sein. Bei einer teilweisen Modifikation werden vorzugsweise endständige Gruppen modifiziert, Oligonukleotide bei denen alle Phosphatgruppen modifiziert sind, sind jedoch am meisten bevorzugt.

Bevorzugte Zuckermodifikationen umfassen den Austausch einer oder mehrerer Ribosereste des Oligonukleotids durch Hexose (Figur 2) oder durch Aminosäuren (Peptidnukleinsäure, PNA, Figur 2).

Modifikationen der Basen umfassen die Verwendung von 5-Propinyl-Uracyl, 5-Propinylcytosin und tricyclischen Cytosinanalogs Phenoxazin.

Die Synthese modifizierter Oligonukleotide sowie weitere geeignete Modifikationsmöglichkeiten sind in der Literatur beschrieben (vgl. beispielsweise E. Uhlmann, A. Peyman, a.a.O.; M.D. Matteucci, R.W. Wagner, a.a.O.).

Darüber hinaus können die erfindungsgemäßen Oligonukleotide durch terminale 3'-3'- und/oder 5'-5'-Internukleotidbindungen gegen einen Abbau durch Exo-Nukleasen geschützt werden (H. Seliger et al., Nucleosides & Nucleotides 10 (1-3), 469-477 (1991)).

Ferner können die erfindungsgemäßen Oligonukleotide zusätzlich durch Gruppen substituiert sein, die die intrazelluläre Aufnahme begünstigen, die *in vivo* oder *in vitro* als Reportergruppen dienen, und/oder Gruppen, die bei der Hybridisierung des Oligoribonukleotids an die Ziel-RNA diese unter Bindung oder Spaltung angreifen.

Beispielhaft für Gruppen, die die intrazelluläre Aufnahme begünstigen, sind lipophile Reste wie Alkylreste, beispielsweise mit 1 bis 18 C-Atomen, Cholesteryl- oder Thiocholesterylgruppen (E. Uhlmann, A. Peyman, a.a.O.) oder Konjugate, die natürliche Carriersysteme ausnutzen, wie z.B. Gallensäure oder Peptide für den entsprechenden Rezeptor (z.B. Rezeptor-vermittelte Endocytose).

Beispiele für Reportergruppen sind fluoreszierende Gruppen (z.B. Acridinyl-, Dansyl-, Fluorescinylgruppen) oder chemilumineszierende Gruppen wie z.B. Acridiniumester-Gruppen.

Beispiele für Oligonukleotid-Konjugate, die an Nukleinsäuren binden und/oder spalten, finden sich in E. Uhlmann, A. Peyman, a.a.O.). Konjugatpartner sind unter anderem Acridin, Psolaren, Chlorethylaminoaryl, Phenanthredin, Azidophenacyl, Azidoproflavin, Phenazin, Phenanthrolin/Cu, Porphyrin/Fe, Benzo[e]pyridoindol, EDTA/Fe (Mergny et al., Science 256 (1992) 1681).

Die Darstellung der erfindungsgemäßen Oligonukleotide erfolgt auf an sich bekannte Weise (vgl. z.B. E. Uhlmann, A. Peyman, a.a.O.). Bevorzugt ist die Synthese an fester Phase unter Zuhilfenahme eines automatischen Synthesegeräts.

Zur Herstellung von Arzneimitteln werden die erfindungsgemäßen Oligonukleotide mit üblichen Trägerstoffen, Hilfsmitteln und/oder Zusatzstoffen kombiniert. Die Oligonukleotide eignen sich zur systemischen, lokalen, subkutanen, intrathekalen und topischen Anwendung und zur Applikation durch Einlauf. Hierzu können sie gelöst in geeigneten Lösungsmitteln, vorzugsweise wäßrigen Lösungen, in Form von Liposomen, als Emulsion oder in fester Form, beispielsweise als Puder oder in mikroverkapselter Form vorliegen.

Die Menge der Oligonukleotide in den Arzneimitteln richtet sich nach der gewünschten Anwendung und wird vorzugsweise so eingestellt, daß eine Applikation von 0,001 bis 100 mg Oligonukleotid pro kg Körpergewicht, vorzugsweise 0,001 bis 10 mg/kg Körpergewicht, besonders bevorzugt 0,01 bis 3 mg/kg Körpergewicht erreicht wird. Die Behandlung erfolgt vorzugsweise durch wiederholte Anwendung über einen Zeitraum von einem Tag bis 6 Wochen, in einer Dosis von vorzugsweise 0,01 bis 3 mg/kg pro Tag.

Die erfindungsgemäßen Oligonukeotide eignen sich zur Behandlung von Krankheitszuständen, die mit einer erhöhten Zellproliferation einhergehen, insbesondere zur Behandlung von benignen und malignen Tumoren, wie Hodentumoren, Lymphomen, Magenkarzinomen, Blasenkarzinomen, Mammakarzinomen, Bronchialkarzinomen, Sarkomen, Nierenkarzinomen, Melanomen, Autoimmunerkrankungen, Narbenbildung, Entzündungen, Allergien, rheumatischen Erkrankungen und Abwehrreaktionen bei Transplantationen.

Ein besonderer Vorteil der erfindungsgemäßen Oligonukleotide ist darin zu sehen, daß sie die Behandlung von Tumoren erlauben, die gegen herkömmliche Chemotherapeutika resistent sind. Solche Resistenzen treten bei unspezifischen Zytostatika wie beispielsweise Vinblastin oder Cisplatin entweder sekundär, d.h. nach mehrfacher Applikation auf, oder sind bei bestimmten Tumoren wie beispielsweise dem Nierenkarzinom bereits primär vorhanden.

Besonders überraschend war die Feststellung, daß die erfindungsgemäßen Oligonukleotide nicht nur das Wachstum von Zellen inhibieren, sondern auch eine cytotoxische Wirkung aufweisen; d.h. zum Absterben der behandelten Tumorzellen führen. Die zytotoxische Wirkung tritt im allgemeinen nach einer Behandlungszeit von etwa 5 bis 12 Tagen ein. Zur vollständigen Abtötung aller proliferierenden Zellen kann eine Behandlungszeit von einigen Wochen erforderlich sein, wobei die Behandlungszeit von Zeiträumen der Nichtbehandlung unterbrochen sein kann.

Im folgenden wird die Erfindung anhand von Ausführungsbeispielen näher erläutert.

### Beispiel 1

### Wirkung auf das Wachstums von RT4-Zellen im Multizellsphäroid-Test

Es wurde die Wirkung erfindungsgemäßer Oligonukleotide auf Blasenkarzinomzellen der Zell-Linie RT4 an Multizellsphäroiden untersucht und mit entsprechenden sense- und missense-Strängen als Kontrolle verglichen.

Hierzu wurden auf bekannte Weise (Uhlmann und Peyman, a.a.O) 2'-Desoxyoligonukleotide mit den folgenden Sequenzen hergestellt:

Alle Oligonukleotide wurden, wenn nicht anders angegeben, in thiolierter Form eingesetzt, d.h. ein Sauerstoffatom der Phosphorsäurereste war durch ein Schwefelatom ersetzt.

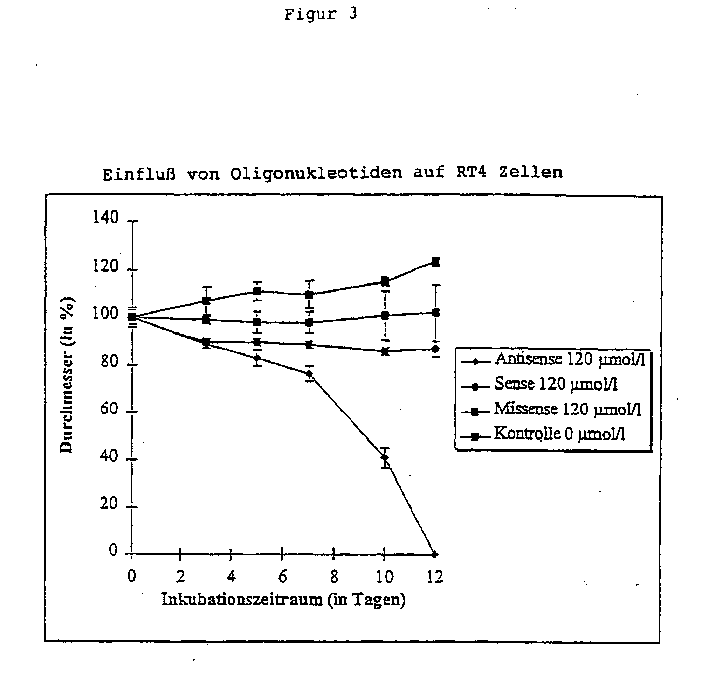

Gemäß der Methode von Carlsson & Yuhas (J. Carlsson und J.M. Yuhas, Liquid-overlay culture of cellular spheroids, Recent Results in Cancer Research 95; 1-23, 1984) wurden Multizellsphäroide der Zell-Linie RT-4 (ATCC Nr.: HTB2) hergestellt. Nach vier Tagen zeigten die Multizellsphäroide eine kugelige Morphologie mit einer ausgeprägten, scharfen Abgrenzung. Anschließend wurden die RT4-Multizellsphäroide in Anwesenheit von 120 µmol/l der jeweiligen Oligonukleotide in Kulturmedien bei 37 °C, 5 % CO₂ inkubiert und die Veränderung des Sphäroiddurchmessers gemessen. Die Oligonukleotide wurden direkt nach der Zeitspanne, die für das Entstehen der Sphäroide notwendig war, in das Medium gegeben. Als Negativkontrollen dienten zum einen eine Probe, der keine Oligonukleotide zugesetzt wurden (Kontrolle), zum anderen die Missense- und Sense-Oligonukleotidproben. Danach wurde der Durchmesser der Multizellsphäroide in Abständen von 2 Tagen gemessen. Pro Test wurden drei gleiche Ansätze untersucht und anschließend der Mittelwert gebildet. Die Ergebnisse sind in Figur 3 graphisch dargestellt.

In der Kontrolle wurde eine Zunahme des Sphäroiddurchmessers auf 132 % des Ausgangswertes beobachtet, während die Zugabe des thiolierten missense-Oligonukleotids einen Wachstumsstillstand bewirkte. Die Zugabe des sense-Oligodesoxynukleotids bewirkte eine geringfügige Verringerung des Sphäroiddurchmessers auf 90 %, während das Antisense-Oligonukleotid zu einer rapiden Abnahme des Sphäroiddurchmessers bis hin zur vollständigen Auflösung des Sphäroids am 12. Tag der Inkubation führte.

Nach der Co-Inkubation der Multizellsphäroide mit Oligonukleotiden wurden diese außerdem unter Zuhilfenahme von Fluoreszenzfarbstoffen in bezug auf ihre Vitalität geprüft. Die dazu verwendeten Farbstoffe waren Fluoreszein-markiertes Dinatriumacetat (FITC-FDA) und Propidium-Iodid (PI). Jedes Multizellsphäroid wurde mit 2 µl FITC-FDA der Konzentration 1 µmol/l für 20 Minuten und mit 10 µl PI (Konzentration: 20 µg/ml) für 10 Minuten inkubiert. Unter dem Fluoreszenzmikroskop erscheinen lebende Zellen durch die FITC-FDA-Färbung grün, tote Zellen durch die PI-Färbung rot. Es zeigte sich eine ausgeprägte zytotoxische Reaktion der untersuchten Zellen in der Antisense-behandelten Gruppe.

Die Ergebnisse zeigen, daß das erfindungsgemäße Antisense-Oligonukleotid für die getestete Tumorzellinie zytotoxisch ist und eine irreversible Zellschädigung bewirkt, die zum Tod der Zelle führt.

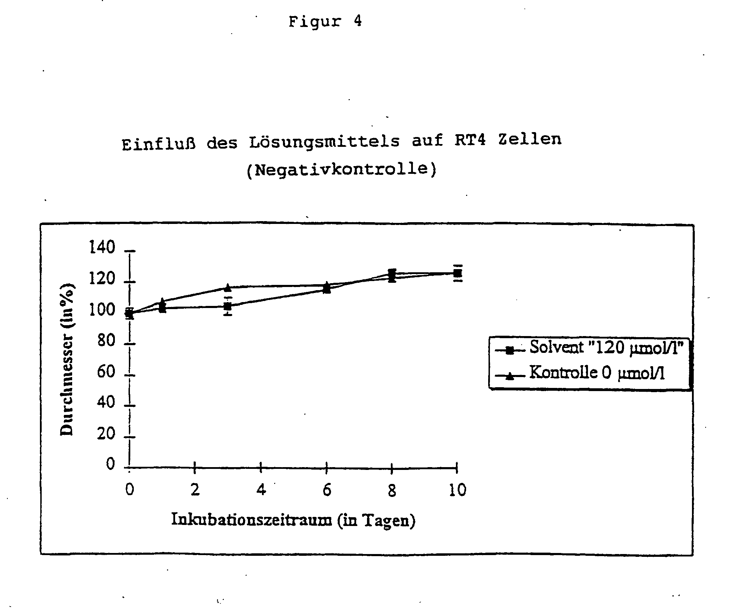

Um auszuschließen, daß das Lösungsmittel allein Einfluß auf das Wachstum besaß, wurden entsprechende Kontrollversuche mit dem Solvens durchgeführt (Solvent; es wurde nur das Lösungsmittel der Oligonukleotide, nicht jedoch die Oligonukeotide selbst zugesetzt), die zeigten, daß diese Einflußgröße zu vernachlässigen war (vgl. Figur 4).

### Beispiel 2

### Wirkung auf das Wachstums von RT4-Zellen bei Mikroinjektion

Es wurde die Wirkung der in Beispiel 1 genannten Oligonukleotide auf RT4-Zellen bei direkter Injektion der Verbindungen in die Zelle untersucht. Hierzu wurden die Oligonukleotide in unmodifizierter (nicht-thiolierter Form) eingesetzt. Durch diesen Test soll einerseits die Wirksamkeit unmodifizierter Oligonukleotide gezeigt und andererseits ausgeschlossen werden, daß eine unspezifische Bindung der Oligodesoxynukleotide an Zellmembranrezeptoren für die in Beispiel 1 beschriebenen Effekte verantwortlich ist.

RT4-Zellen wurden auf speziellen Deckgläsern (CELLocate® -Deckgläser, Fa. Eppendorf) ausgesät. Bei diesen Deckgläsern erleichtert ein in der Mitte eingeätztes Raster das Wiederfinden der injizierten Zellen. Vor der Aussaat der Zellen wurden die Deckgläser in Petrischalen mit einem Durchmesser von 3,5 cm plaziert und mit je 1 µl Fibronektin benetzt, das für ein besseres Anheften der Zellen sorgt. Pro Schale wurden dann 1.5 × 10⁵ Zellen, die vorher mittels Trypsin gelöst worden waren, in 2,5 ml supplierten RPMI 1640 Medium ausgesät und bei 37 °C über Nacht im Brutschrank kultiviert.

Die Mikroinjektion wurde mit Hilfe des Transjektors 5246 und des Mikromanipulators 5171 (Firma Eppendorf) unter lichtmikroskopischer Kontrolle durchgeführt (inverses Mikroskop Typ Leitz DMIL, Firma Leica). Die Mikroinjektionskapillaren wurden mit Hilfe von Mikroloader® Pipettenspitzen (Fa. Eppendorf) mit jeweils 2,0 µl Oligonukleotidlösung (Konzentration 120 µmol/l) befüllt. Die Konzentrationseinstellung erfolgte mit steril filtriertem phosphatgepufferter Salzlösung (phosphate buffered saline, PBS). Um die Durchlässigkeit der befüllten Kapillare zu überprüfen, wurde die Clean-Funktion des Transjektors unter mikroskopischer Kontrolle eingesetzt. Bei offener Kapillare war nach dem Eintauchen in das Kulturmedium ein gleichmäßiger Ausstrom an Injektionsflüssigkeit zu beobachten. Der Injektionsdruck wurde empirisch auf 130 hPa festgelegt und nach den ersten Injektionen so korrigiert, daß die Injektion zu einer deutlichen Größenzunahme der Zelle führte, ohne sie zu zerstören. Die Injektionszeit betrug zwischen 0,3 und 0,5 Sekunden.

Für zytoplasmatische Injektionen wurde die Kapillarspitze solange an das Zytoplasma herangeführt, bis ein druckbedingter Lichtreflex an der Zelle zu beobachten war. Dann wurde die Kapillare wieder um einige µm angehoben und mittels Knopfdruck die automatische Injektionsbewegung ausgelöst. Während der Injektion konnte das Injektionslimit in 0,14 µm Schritten nach oben oder unten korrigiert werden, so daß Unebenheiten des Zelluntergrundes ausgeglichen werden konnten. Für vergleichende Untersuchungen wurden Mikroinjektionskapillaren verwendet, die in einem Arbeitsgang gezogen wurden, um bei gleichen Injektionsparametern die ausströmende Flüssigkeitsmenge pro Injektion möglichst konstant zu halten. Dennoch variierte das initiierte Volumen von Zelle zu Zelle, da sich der Injektionsdruck- und damit die zu injizierende Lösung, je nach getroffenem Bereich besser oder schlechter ausbreiten konnte. Um die Auswirkungen von Auskühlung und pH-Verschiebung des Kulturmediums auf das Wachstumsverhalten der Zellen zu minimieren, wurde die Gesamtinjektionszeit pro Zellkulturschälchen auf 15 Minuten beschränkt.

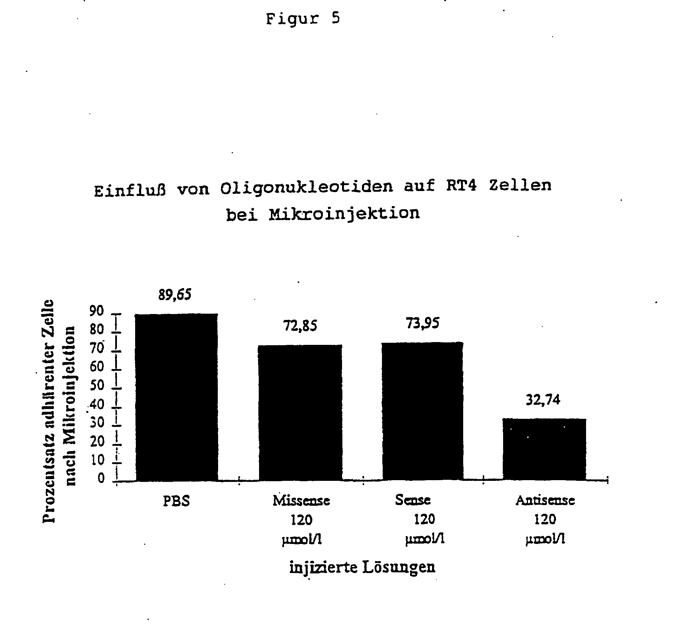

Die Ergebnisse des Tests sind in Fig. 5 graphisch dargestellt. Es zeigte sich, daß die Injektion von Antisense-Oligonukleotiden und nachfolgende 22stündiger Inkubationszeit zu einem Verlust der Adhäsion bei ca. 70 % der Zellen zur Folge hatte. Da nur lebende Zellen am Deckglas haften bleiben, ist dieses Ergebnis mit einem Absterben von 70 % der Zellen gleichzusetzen. Die Injektion des sense- oder missense-Oligonukleotids führte lediglich bei jeweils 30 % der Zellen und die alleinige Injektion des Lösungsmittels (PBS) bei 10 % der Zellen zum Adhäsionsverlust.

### Beispiel 3

### Wirkung auf das Wachstum von J82-Zellen

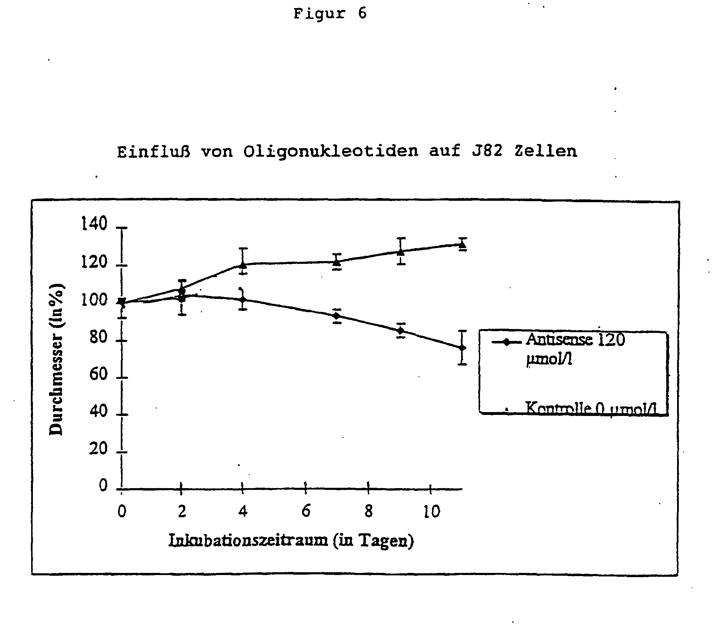

Analog zu Beispiel 1 wurde die Wirkung der Oligonukleotide auf die humane Blasentumorzellinie J82 untersucht. Das thiolierte Antisense-Oligonukleotid führte in einer Konzentration von 120 µmol/l nach 11 Tagen zu einer Abnahme des Sphäroiddurchmessers um 20 %, während der Sphäroiddurchmesser der Kontrolle im gleichen Zeitraum um etwa 30 % zunahm (Fig. 6).

## Patentansprüche

1. Verwendung eines Oligoribo- oder Oligodesoxyribonukleotids, das in der Lage ist, mit der mRNA zu hybridisieren, die für das Protein Ki-67 kodiert, oder eines physiologisch verträglichen Salzes davon, zur Herstellung eines Arzneimittels zum Abtöten von proliferierenden Zellen.

2. Verwendung nach Anspruch 1, **dadurch gekennzeichnet, daß** die Nukleotidsequenz des Oligoribo- oder Oligodesoxyribonukleotids zu der SEQ ID NO 1 komplementär ist.

3. Verwendung nach Anspruch 2, **dadurch gekennzeichnet, daß** die Nukleotidsequenz des Oligoribo- oder Oligodesoxyribonukleotids zu dem Abschnitt von Position 197 bis 9962 der SEQ ID NO 1 komplementär ist.

4. Verwendung nach einem der Ansprüche 1 bis 3, **dadurch gekennzeichnet, dass** das Oligoribo- oder Oligodesoxyribonukleotid 12 bis 66 Nukleotide aufweist.

5. Verwendung nach Ansprüche 1 bis 4, **dadurch gekennzeichnet, dass** das Oligoribo- oder Oligodesoxyribonukleotid 17 bis 46 Nukleotide aufweist.

6. Verwendung nach einem der Ansprüche 1 bis 5, **dadurch gekennzeichnet, daß** das Oligoribo- oder Oligodesoxyribonukleotid die Sequenz (5'-ACC AGG CGT CTC GTG GGC CAC AT) aufweist.

7. Verwendung nach einem der Ansprüche 1 bis 6, **dadurch gekennzeichnet, daß** eine oder mehrere Phosphatgruppen des Oligoribo- oder Oligodesoxyribonukleotids durch Phosphothioat-, Methylphosphonat-, Phosphoramidat-, Methylen-(methylimino)- und/oder Guanidingruppe(n) ausgetauscht sind.

8. Verwendung nach einem der Ansprüche 1 bis 7, **dadurch gekennzeichnet, daß** das Oligoribo- oder Oligodesoxyribonukleotid eine terminale 3'-3'- und/oder 5'-5'-Internukleotidverknüpfung aufweist.

9. Arzneimittel zum Abtöten von proliferierenden Zellen, **gekennzeichnet durch** einen Gehalt an einem Oligoribo- und/oder Oligodesoxyribonukleotid, das in der Lage ist, mit der mRNA zu hybridisieren, die für das zellcyclusassoziierte Protein Ki-67 kodiert, oder einem physiologisch verträglichen Salze davon, neben üblichen Trägerstoffen, Hilfsmitteln und/oder Zusatzstoffen, wobei die Oligonukleotid-Menge so eingestellt ist, dass eine Applikation von 0,001 bis 100 mg/kg Körpergewicht erzielt wird.

10. Verwendung eines Oligoribo- oder Oligodesoxyribonucleotids nach einem der Ansprüche 1 bis 8 zum Herstellen eines Medikamentes zum Abtöten von proliferierenden Zellen bei der Behandlung von Tumoren, Lymphomen, Autoimmunerkrankungen, Narbenbildung, Entzündungen, Allergien, rheumatischen Erkrankungen, Abwehrreaktionen nach Transplantationen.

11. Verfahren zur Herstellen eines Arzneimittels gemäss Anspruch 9 zum Abtöten von proliferierenden Zellen, **gekennzeichnet durch** die Verwendung von Oligoribo- oder Oligodesoxyribonukfeotiden, die in der Lage sind, mit der mRNA zu hybridisieren, die für das Protein Ki-67 kodiert, oder eines physiologisch verträglichen Salzes.

12. Verfahren nach Anspruch 11 zur Herstellung eines Arzneimittels zur Behandlung von Tumoren, Autoimmunerkrankungen, Narbenbildung, Entzündungen, Allergien, rheumatischen Erkrankungen oder Abwehrreaktionen nach Transplantationen.

13. Verfahren nach Anspruch 11 oder 12 umfassend das Kombinieren eines Oligoribo- oder Oligodesoxyribonukleotids, das in der Lage ist, mit der mRNA zu hybridisieren, die für das Protein Ki-67 kodiert, mit üblichen Trägerstoffen, Hilfsmitteln und/oder Zusatzstoffen.

14. Oligoribo- oder Oligodesoxyribonukleotid oder ein physiologisch verträgliches Salz davon, **dadurch gekennzeichnet, dass** es in der Lage ist, mit der mRNA zu hybridisieren, die für das Protein Ki -67 kodiert, dass es 22 bis 46 Nukleotide aufweist, und dass es eine zytotoxische Wirkung auf proliferierende Zellen hat.

15. Oligoribo- oder Oligodesoxyribonukleotid nach Anspruch 14, **dadurch gekennzeichnet, dass** es die Sequenz (5'-ACC AGG CGT CTC GTG GGC CAC AT) aufweist

## Claims

1. Use of an oligoribo- or oligodeoxyribonucleotide which is capable of hybridizing with the mRNA which codes for the protein Ki-67, or of a physiologically acceptable salt thereof, for preparing a medicament for killing proliferating cells.

2. Use according to claim 1, **characterized in that** the nucleotide sequence of the oligoribo- or oligodeoxyribonucleotide is complementary to SEQ ID NO 1.

3. Use according to claim 2, **characterized in that** the nucleotide sequence of the oligoribo- or oligodeoxyribonucleotide is complementary to the section from position 197 to 9962 of SEQ ID NO 1.

4. Use according to any one of claims 1 to 3, **characterized in that** the oligoribo- or oligodeoxyribonucleotide comprises 12 to 66 nucleotides.

5. Use according to any one of claims 1 to 4, **characterized in that** the oligoribo- or oligodeoxyribonucleotide comprises 17 to 46 nucleotides.

6. Use according to any one of claims 1 to 5, **characterized in that** the oligoribo- or oligodeoxyribonucleotide has the sequence (5'-ACC AGG CGT CTC GTG GGC CAC AT).

7. Use according to any one of claims 1 to 6, **characterized in that** one or more phosphate groups of the oligoribo- or oligodeoxyribonucleotide are replaced by phosphothioate, methylphosphonate, phosphoramidate, methylene(methylimino) and/or guanidine group(s).

8. Use according to any one of claims 1 to 7, **characterized in that** the oligoribo- or oligodeoxyribonucleotide has a terminal 3'-3' and/or 5'-5' internucleotide linkage.

9. A medicament for killing proliferating cells, **characterized by** a content of an oligoribo- and/or oligodeoxyribonucleotide which is capable of hybridizing with the mRNA which codes for the cell cycle-associated protein Ki-67, or of a physiologically acceptable salt thereof, in addition to conventional carrier substances, auxiliaries and/or additives, the oligonucleotide amount being adjusted such that an administration of 0.001 to 100 mg/kg of body weight is achieved.

10. Use of an oligoribo- or oligodeoxyribonucleotide according to any one of claims 1 to 8 for preparing a medicament for killing proliferating cells in the treatment of tumors, lymphomas, autoimmune disease, cicatrisation, inflammations, allergies, rheumatic diseases, rejection reactions following transplantations.

11. A method for preparing a medicament according to claim 9 for killing proliferating cells, **characterized by** the use of oligoribo- or oligodeoxyribonucleotides which are capable of hybridizing with the mRNA which codes for the protein Ki-67, or of a physiologically acceptable salt.

12. A method according to claim 11 for preparing a medicament for the treatment of tumors, autoimmune diseases, cicatrisation, inflammations, allergies, rheumatic diseases or rejection reactions following transplantations.

13. A method according to claim 11 or 12, comprising combining an oligoribo- or oligodeoxyribonucleotide which is capable of hybridizing with the mRNA which codes for the protein Ki-67, with conventional carrier substances, auxiliaries and/or additives.

14. Oligoribo- or oligodeoxyribonucleotide or a physiologically acceptable salt thereof, **characterized in that** it is capable of hybridizing with the mRNA which codes for the protein Ki-67, that it comprises 22 to 46 nucleotides, and that it has a cytotoxic effect on proliferating cells.

15. Oligoribo- or oligodeoxyribonucleotide according to claim 14, **characterized in that** it has the sequence (5'-ACC AGG CGT CTC GTG GGC CAC AT).

## Revendications

1. Utilisation d'un oligoribo- ou d'un oligodésoxyribonucléotide, qui est à même de s'hybrider à l'ARNm qui code pour la protéine Ki-67, ou d'un sel physiologiquement compatible de celui-ci, pour préparer un médicament destiné à la destruction de cellules proliférantes.

2. Utilisation selon la revendication 1, **caractérisée en ce que** la séquence nucléotidique de l'oligoribo- ou de l'oligodésoxyribonucléotide est complémentaire de la séquence SEQ ID NO 1.

3. Utilisation selon la revendication 2, **caractérisée en ce que** la séquence nucléotidique de l'oligoribo- ou de l'oligodésoxyribonucléotide est complémentaire du segment allant de la position 197 à la position 9962 de la séquence SEQ ID NO 1.

4. Utilisation selon l'une des revendications 1 à 3, **caractérisée en ce que** l'oligoribo- ou l'oligodésoxyribo- nucléotide comprend de 12 à 66 nucléotides.

5. Utilisation selon les revendications 1 à 4, **caractérisée en ce que** l'oligoribo- ou l'oligodésoxyribo- nucléotide comprend de 17 à 46 nucléotides.

6. Utilisation selon l'une des revendications 1 à 5, **caractérisée en ce que** l'oligoribo- ou l'oligodésoxyribo- nucléotide comprend la séquence (5'-ACC AGG CGT CTC GTG GGC CAC AT).

7. Utilisation selon l'une des revendications 1 à 6, **caractérisée en ce qu'**un ou plusieurs groupes phosphate de l'oligoribo- ou de l'oligodésoxyribonucléotide sont remplacés par un ou des groupes phosphothioate, méthylphosphonate, phosphoramidate, méthylène (méthylimino) et/ou guanidine.

8. Utilisation selon l'une des revendications 1 à 7, **caractérisée en ce que** l'oligoribo- ou l'oligodésoxyribo- nucléotide comprend une liaison 3'-3'- et/ou 5'-5'-internucléotidique terminale.

9. Médicament pour détruire des cellules proliférantes, **caractérisé en ce qu'**il contient un oligoribo- et/ou un oligodésoxyribonucléotide qui est à même de s'hybrider avec l'ARNm qui code pour la protéine associée au cycle cellulaire Ki-67, ou un sel physiologiquement compatible de celui-ci, outre des supports, adjuvants et/ou additifs usuels, la quantité de l'oligonucléotide étant ajustée de façon à parvenir à l'administration de 0,001 à 100 mg/kg de poids corporel.

10. Utilisation d'un oligoribo- ou d'un oligodésoxyribonucléotide selon l'une des revendications 1 à 8 pour fabriquer un médicament destiné à détruire des cellules proliférantes lors du traitement de tumeurs, de lymphomes, de maladies auto-immunes, de cicatrisation, d'inflammations, d'allergies, de maladies rhumatismales, de réactions de défense après des transplantations.

11. Procédé de fabrication d'un médicament selon la revendication 9 pour détruire des cellules proliférantes, **caractérisé par** l'utilisation d'oligoribo- ou d'oligodésoxyribonucléotides qui sont à même de s'hybrider avec l'ARNm qui code pour la protéine Ki-67, ou d'un sel physiologiquement compatible.

12. Procédé selon la revendication 11 pour fabriquer un médicament destiné au traitement de tumeurs, de maladies auto-immunes, de cicatrisation, d'inflammations, d'allergies, de maladies rhumatismales ou de réactions de défense après des transplantations.

13. Procédé selon la revendication 11 ou 12, qui comprend la combinaison d'un oligoribo- ou d'un oligodésoxyribonucléotide qui est à même de s'hybrider avec l'ARNm qui code pour la protéine Ki-67, avec des supports, adjuvants et/ou additifs usuels.

14. Oligoribo- ou oligodésoxyribonucléotide, ou sel physiologiquement compatible de celui-ci, **caractérisé en ce qu'**il est à même de s'hybrider avec l'ARNm qui code pour la protéine Ki-67, qu'il comporte de 22 à 46 nucléotides, et qu'il a un effet cytotoxique sur les cellules proliférantes.

15. Oligoribo- ou oligodésoxyribonucléotide selon la revendication 14, **caractérisé en ce qu'**il comporte la séquence (5'-ACC AGG CGT CTC GTG GGC CAC AT).


### SEQUENZ PROTOKOLL

(1) ALLGEMEINE ANGABEN:
   (i) ANMELDER:
      (A) NAME: Forschungszentrum Borstel
      (B) STRASSE: Parkallee 1-40
      (C) ORT: Borstel
      (D) BUNDESLAND: Schleswig-Holstein
      (E) LAND: Deutschland
      (F) POSTLEITZAHL: D 23845
   (ii) BEZEICHNUNG DER ERFINDUNG: Antisense-Oligonukleotide zur Behandlung von proliferierenden Zellen
   (iii) ANZAHL DER SEQUENZEN: 3
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 12493 Basenpaare
      (B) ART: Nucleotid
      (C) STRANGFORM: Doppelstrang
      (D) TOPOLOGIE: linear
   (ii) ART DES MOLEKÜLS: cDNS
   (ix) MERKMAL:
      (A) NAME/SCHLÜSSEL: CDS
      (B) LAGE:197..9964
   (xi) SEQUENZBESCHREIBUNG: SEQ ID NO: 1:
   CTACCGGGCG GAGGTGAGCG CGGCGCCGGC TCCTCCTGCG GCGGACTTTG GGTGCGACTT      60
   GACGAGCGGT GGTTCGACAA GTGGCCTTGC GGGCCGGATC GTCCCAGTGG AAGAGTTGTA     120
   AATTTGCTTC TGGCCTTCCC CTACGGATTA TACCTGGCCT TCCCCTACGG ATTATACTCA     180
   ACTTACTGTT TAGAAA ATG TGG CCC ACG AGA CGC CTG GTT ACT ATC AAA         229
                     Met Trp Pro Thr Arg Arg Leu Val Thr Ile Lys
                      1               5                  10
   AGG AGC GGG GTC GAC GGT CCC CAC TTT CCC CTG AGC CTC AGC ACC TGC       277
   Arg Ser Gly Val Asp Gly Pro His Phe Pro Leu Ser Leu Ser Thr Cys
               15                  20                  25
   TTG TTT GGA AGG GGT ATT GAA TGT GAC ATC CGT ATC CAG CTT CCT GTT       325
   Leu Phe Gly Arg Gly Ile Glu Cys Asp Ile Arg Ile Gln Leu Pro Val
           30                  35                  40
   GTG TCA AAA CAA CAT TGC AAA GTT GAA ATC CAT GAG CAG GAG GCA ATA       373
   Val Ser Lys Gln His Cys Lys Val Glu Ile His Glu Gln Glu Ala Ile
       45                  50                  55
   TTA CAT AAT TTC AGT TCC ACA AAT CCA ACA CAA GTA AAT GGG TCT GTT    421
   Leu His Asn Phe Ser Ser Thr Asn Pro Thr Gln Val Asn Gly Ser Val
    60                 65                  70                  75
   ATT GAT GAG CCT GTA CGG CTA AAA CAT GGA GAT GTA ATA ACT ATT ATT    469
   Ile Asp Glu Pro Val Arg Leu Lys His Gly Asp Val Ile Thr Ile Ile
                    80                  85                  90
   GAT CGT TCC TTC AGG TAT GAA AAT GAA AGT CTT CAG AAT GGA AGG AAG    517
   Asp Arg Ser Phe Arg Tyr Glu Asn Glu Ser Leu Gln Asn Gly Arg Lys
                95                 100                 105
   TCA ACT GAA TTT CCA AGA AAA ATA CGT GAA CAG GAG CCA GCA CGT CGT    565
   Ser Thr Glu Phe Pro Arg Lys Ile Arg Glu Gln Glu Pro Ala Arg Arg
           110                 115                 120
   GTC TCA AGA TCT AGC TTC TCT TCT GAC CCT GAT GAG AAA GCT CAA GAT    613
   Val Ser Arg Ser Ser Phe Ser Ser Asp Pro Asp Glu Lys Ala Gln Asp
       125                 130                 135
   TCC AAG GCC TAT TCA AAA ATC ACT GAA GGA AAA GTT TCA GGA AAT CCT    661
   Ser Lys Ala Tyr Ser Lys Ile Thr Glu Gly Lys Val Ser Gly Asn Pro
   140                 145                 150                 155
   CAG GTA CAT ATC AAG AAT GTC AAA GAA GAC AGT ACC GCA GAT GAC TCA    709
   Gln Val His Ile Lys Asn Val Lys Glu Asp Ser Thr Ala Asp Asp Ser
                   160                 165                 170
   AAA GAC AGT GTT GCT CAG GGA ACA ACT AAT GTT CAT TCC TCA GAA CAT    757
   Lys Asp Ser Val Ala Gln Gly Thr Thr Asn Val His Ser Ser Glu His
               175                 180                 185
   GCT GGA CGT AAT GGC AGA AAT GCA GCT GAT CCC ATT TCT GGG GAT TTT    805
   Ala Gly Arg Asn Gly Arg Asn Ala Ala Asp Pro Ile Ser Gly Asp Phe
           190                 195                 200
   AAA GAA ATT TCC AGC GTT AAA TTA GTG AGC CGT TAT GGA GAA TTG AAG    853
   Lys Glu Ile Ser Ser Val Lys Leu Val Ser Arg Tyr Gly Glu Leu Lys
       205                 210                 215
   TCT GTT CCC ACT ACA CAA TGT CTT GAC AAT AGC AAA AAA AAT GAA TCT    901
   Ser Val Pro Thr Thr Gln Cys Leu Asp Asn Ser Lys Lys Asn Glu Ser
   220                 225                 230                 235
   CCC TTT TGG AAG CTT TAT GAG TCA GTG AAG AAA GAG TTG GAT GTA AAA    949
   Pro Phe Trp Lys Leu Tyr Glu Ser Val Lys Lys Glu Leu Asp Val Lys
                   240                 245                 250
   TCA CAA AAA GAA AAT GTC CTA CAG TAT TGT AGA AAA TCT GGA TTA CAA    997
   Ser Gln Lys Glu Asn Val Leu Gln Tyr Cys Arg Lys Ser Gly Leu Gln
               255                 260                 265
   ACT GAT TAC GCA ACA GAG AAA GAA AGT GCT GAT GGT TTA CAG GGG GAG   1045
   Thr Asp Tyr Ala Thr Glu Lys Glu Ser Ala Asp Gly Leu Gln Gly Glu
           270                 275                 280
   ACC CAA CTG TTG GTC TCG CGT AAG TCA AGA CCA AAA TCT GGT GGG AGC   1093
   Thr Gln Leu Leu Val Ser Arg Lys Ser Arg Pro Lys Ser Gly Gly Ser
       285                 290                 295
   GGC CAC GCT GTG GCA GAG CCT GCT TCA CCT GAA CAA GAG CTT GAC CAG   1141
   Gly His Ala Val Ala Glu Pro Ala Ser Pro Glu Gln Glu Leu Asp Gln
   300                 305                 310                 315
   AAC AAG GGG AAG GGA AGA GAC GTG GAG TCT GTT CAG ACT CCC AGC AAG      1189
   Asn Lys Gly Lys Gly Arg Asp Val Glu Ser Val Gln Thr Pro Ser Lys
                   320                 325                 330
   GCT GTG GGC GCC AGC TTT CCT CTC TAT GAG CCG GCT AAA ATG AAG ACC      1237
   Ala Val Gly Ala Ser Phe Pro Leu Tyr Glu Pro Ala Lys Met Lys Thr
               335                 340                 345
   CCT GTA CAA TAT TCA CAG CAA CAA AAT TCT CCA CAA AAA CAT AAG AAC      1285
   Pro Val Gln Tyr Ser Gln Gln Gln Asn Ser Pro Gln Lys His Lys Asn
           350                 355                 360
   AAA GAC CTG TAT ACT ACT GGT AGA AGA GAA TCT GTG AAT CTG GGT AAA      1333
   Lys Asp Leu Tyr Thr Thr Gly Arg Arg Glu Ser Val Asn Leu Gly Lys
       365                 370                 375
   AGT GAA GGC TTC AAG GCT GGT GAT AAA ACT CTT ACT CCC AGG AAG CTT      1381
   Ser Glu Gly Phe Lys Ala Gly Asp Lys Thr Leu Thr Pro Arg Lys Leu
   380                 385                 390                 395
   TCA ACT AGA AAT CGA ACA CCA GCT AAA GTT GAA GAT GCA GCT GAC TCT      1429
   Ser Thr Arg Asn Arg Thr Pro Ala Lys Val Glu Asp Ala Ala Asp Ser
                   400                 405                 410
   GCC ACT AAG CCA GAA AAT CTC TCT TCC AAA ACC AGA GGA AGT ATT CCT      1477
   Ala Thr Lys Pro Glu Asn Leu Ser Ser Lys Thr Arg Gly Ser Ile Pro
               415                 420                 425
   ACA GAT GTG GAA GTT CTG CCT ACG GAA ACT GAA ATT CAC AAT GAG CCA      1525
   Thr Asp Val Glu Val Leu Pro Thr Glu Thr Glu Ile His Asn Glu Pro
           430                 435                 440
   TTT TTA ACT CTG TGG CTC ACT CAA GTT GAG AGG AAG ATC CAA AAG GAT      1573
   Phe Leu Thr Leu Trp Leu Thr Gln Val Glu Arg Lys Ile Gln Lys Asp
       445                 450                 455
   TCC CTC AGC AAG CCT GAG AAA TTG GGC ACT ACA GCT GGA CAG ATG TGC      1621
   Ser Leu Ser Lys Pro Glu Lys Leu Gly Thr Thr Ala Gly Gln Met Cys
   460                 465                 470                 475
   TCT GGG TTA CCT GGT CTT AGT TCA GTT GAT ATC AAC AAC TTT GGT GAT      1669
   Ser Gly Leu Pro Gly Leu Ser Ser Val Asp Ile Asn Asn Phe Gly Asp
                   480                 485                 490
   TCC ATT AAT GAG AGT GAG GGA ATA CCT TTG AAA AGA AGG CGT GTG TCC      1717
   Ser Ile Asn Glu Ser Glu Gly Ile Pro Leu Lys Arg Arg Arg Val Ser
               495                 500                 505
   TTT GGT GGG CAC CTA AGA CCT GAA CTA TTT GAT GAA AAC TTG CCT CCT      1765
   Phe Gly Gly His Leu Arg Pro Glu Leu Phe Asp Glu Asn Leu Pro Pro
           510                 515                 520
   AAT ACG CCT CTC AAA AGG GGA GAA GCC CCA ACC AAA AGA AAG TCT CTG      1813
   Asn Thr Pro Leu Lys Arg Gly Glu Ala Pro Thr Lys Arg Lys Ser Leu
       525                 530                 535
   GTA ATG CAC ACT CCA CCT GTC CTG AAG AAA ATC ATC AAG GAA CAG CCT      1861
   Val Met His Thr Pro Pro Val Leu Lys Lys Ile Ile Lys Glu Gln Pro
   540                 545                 550                 555
   CAA CCA TCA GGA AAA CAA GAG TCA GGT TCA GAA ATC CAT GTG GAA GTG      1909
   Gln Pro Ser Gly Lys Gln Glu Ser Gly Ser Glu Ile His Val Glu Val
                   560                 565                 570
   AAG GCA CAA AGC TTG GTT ATA AGC CCT CCA GCT CCT AGT CCT AGG AAA 1957
   Lys Ala Gln Ser Leu Val Ile Ser Pro Pro Ala Pro Ser Pro Arg Lys
   575 580 585
   ACT CCA GTT GCC AGT GAT CAA CGC CGT AGG TCC TGC AAA ACA GCC CCT 2005
   Thr Pro Val Ala Ser Asp Gln Arg Arg Arg Ser Cys Lys Thr Ala Pro
   590 595 600
   GCT TCC AGC AGC AAA TCT CAG ACA GAG GTT CCT AAG AGA GGA GGA GAA 2053
   Ala Ser Ser Ser Lys Ser Gln Thr Glu Val Pro Lys Arg Gly Gly Glu
   605 610 615
   AGA GTG GCA ACC TGC CTT CAA AAG AGA GTG TCT ATC AGC CGA AGT CAA 2101
   Arg Val Ala Thr Cys Leu Gln Lys Arg Val Ser Ile Ser Arg Ser Gln
   620 625 630 635
   CAT GAT ATT TTA CAG ATG ATA TGT TCC AAA AGA AGA AGT GGT GCT TCG 2149
   His Asp Ile Leu Gln Met Ile Cys Ser Lys Arg Arg Ser Gly Ala Ser
   640 645 650
   GAA GCA AAT CTG ATT GTT GCA AAA TCA TGG GCA GAT GTA GTA AAA CTT 2197
   Glu Ala Asn Leu Ile Val Ala Lys Ser Trp Ala Asp Val Val Lys Leu
   655 660 665
   GGT GCA AAA CAA ACA CAA ACT AAA GTC ATA AAA CAT GGT CCT CAA AGG 2245
   Gly Ala Lys Gln Thr Gln Thr Lys Val Ile Lys His Gly Pro Gln Arg
   670 675 680
   TCA ATG AAC AAA AGG CAA AGA AGA CCT GCT ACT CCA AAG AAG CCT GTG 2293
   Ser Met Asn Lys Arg Gln Arg Arg Pro Ala Thr Pro Lys Lys Pro Val
   685 690 695
   GGC GAA GTT CAC AGT CAA TTT AGT ACA GGC CAC GCA AAC TCT CCT TGT 2341
   Gly Glu Val His Ser Gln Phe Ser Thr Gly His Ala Asn Ser Pro Cys
   700 705 710 715
   ACC ATA ATA ATA GGG AAA GCT CAT ACT GAA AAA GTA CAT GTG CCT GCT 2389
   Thr Ile Ile Ile Gly Lys Ala His Thr Glu Lys Val His Val Pro Ala
   720 725 730
   CGA CCC TAC AGA GTG CTC AAC AAC TTC ATT TCC AAC CAA AAA ATG GAC 2437
   Arg Pro Tyr Arg Val Leu Asn Asn Phe Ile Ser Asn Gln Lys Met Asp
   735 740 745
   TTT AAG GAA GAT CTT TCA GGA ATA GCT GAA ATG TTC AAG ACC CCA GTG 2485
   Phe Lys Glu Asp Leu Ser Gly Ile Ala Glu Met Phe Lys Thr Pro Val
   750 755 760
   AAG GAG CAA CCG CAG TTG ACA AGC ACA TGT CAC ATC GCT ATT TCA AAT 2533
   Lys Glu Gln Pro Gln Leu Thr Ser Thr Cys His Ile Ala Ile Ser Asn
   765 770 775
   TCA GAG AAT TTG CTT GGA AAA CAG TTT CAA GGA ACT GAT TCA GGA GAA 2581
   Ser Glu Asn Leu Leu Gly Lys Gln Phe Gln Gly Thr Asp Ser Gly Glu
   780 785 790 795
   GAA CCT CTG CTC CCC ACC TCA GAG AGT TTT GGA GGA AAT GTG TTC TTC 2629
   Glu Pro Leu Leu Pro Thr Ser Glu Ser Phe Gly Gly Asn Val Phe Phe
   800 805 810
   AGT GCA CAG AAT GCA GCA AAA CAG CCA TCT GAT AAA TGC TCT GCA AGC 2677
   Ser Ala Gln Asn Ala Ala Lys Gln Pro Ser Asp Lys Cys Ser Ala Ser
   815 820 825
   CCT CCC TTA AGA CGG CAG TGT ATT AGA GAA AAT GGA AAC GTA GCA AAA        2725
   Pro Pro Leu Arg Arg Gln Cys Ile Arg Glu Asn Gly Asn Val Ala Lys
           830                 835                 840
   ACG CCC AGG AAC ACC TAC AAA ATG ACT TCT CTG GAG ACA AAA ACT TCA        2773
   Thr Pro Arg Asn Thr Tyr Lys Met Thr Ser Leu Glu Thr Lys Thr Ser
       845                 850                 855
   GAT ACT GAG ACA GAG CCT TCA AAA ACA GTA TCC ACT GTA AAC AGG TCA        2821
   Asp Thr Glu Thr Glu Pro Ser Lys Thr Val Ser Thr Val Asn Arg Ser
   860                 865                 870                 875
   GGA AGG TCT ACA GAG TTC AGG AAT ATA CAG AAG CTA CCT GTG GAA AGT        2869
   Gly Arg Ser Thr Glu Phe Arg Asn Ile Gln Lys Leu Pro Val Glu Ser
                   880                 885                 890
   AAG AGT GAA GAA ACA AAT ACA GAA ATT GTT GAG TGC ATC CTA AAA AGA        2917
   Lys Ser Glu Glu Thr Asn Thr Glu Ile Val Glu Cys Ile Leu Lys Arg
               895                 900                 905
   GGT CAG AAG GCA ACA CTA CTA CAA CAA AGG AGA GAA GGA GAG ATG AAG        2965
   Gly Gln Lys Ala Thr Leu Leu Gln Gln Arg Arg Glu Gly Glu Met Lys
           910                 915                 920
   GAA ATA GAA AGA CCT TTT GAG ACA TAT AAG GAA AAT ATT GAA TTA AAA        3013
   Glu Ile Glu Arg Pro Phe Glu Thr Tyr Lys Glu Asn Ile Glu Leu Lys
       925                 930                 935
   GAA AAC GAT GAA AAG ATG AAA GCA ATG AAG AGA TCA AGA ACT TGG GGG        3061
   Glu Asn Asp Glu Lys Met Lys Ala Met Lys Arg Ser Arg Thr Trp Gly
   940                 945                 950                 955
   CAG AAA TGT GCA CCA ATG TCT GAC CTG ACA GAC CTC AAG AGC TTG CCT        3109
   Gln Lys Cys Ala Pro Met Ser Asp Leu Thr Asp Leu Lys Ser Leu Pro
                   960                 965                 970
   GAT ACA GAA CTC ATG AAA GAC ACG GCA CGT GGC CAG AAT CTC CTC CAA        3157
   Asp Thr Glu Leu Met Lys Asp Thr Ala Arg Gly Gln Asn Leu Leu Gln
               975                 980                 985
   ACC CAA GAT CAT GCC AAG GCA CCA AAG AGT GAG AAA GGC AAA ATC ACT        3205
   Thr Gln Asp His Ala Lys Ala Pro Lys Ser Glu Lys Gly Lys Ile Thr
           990                 995                 1000
   AAA ATG CCC TGC CAG TCA TTA CAA CCA GAA CCA ATA AAC ACC CCA ACA        3253
   Lys Met Pro Cys Gln Ser Leu Gln Pro Glu Pro Ile Asn Thr Pro Thr
       1005                1010                1015
   CAC ACA AAA CAA CAG TTG AAG GCA TCC CTG GGG AAA GTA GGT GTG AAA        3301
   His Thr Lys Gln Gln Leu Lys Ala Ser Leu Gly Lys Val Gly Val Lys
   1020                1025                1030                1035
   GAA GAG CTC CTA GCA GTC GGC AAG TTC ACA CGG ACG TCA GGG GAG ACC        3349
   Glu Glu Leu Leu Ala Val Gly Lys Phe Thr Arg Thr Ser Gly Glu Thr
                   1040                1045                1050
   ACG CAC ACG CAC AGA GAG CCA GCA GGA GAT GGC AAG AGC ATC AGA ACG        3397
   Thr His Thr His Arg Glu Pro Ala Gly Asp Gly Lys Ser Ile Arg Thr
               1055                1060                1065
   TTT AAG GAG TCT CCA AAG CAG ATC CTG GAC CCA GCA GCC CGT GTA ACT        3445
   Phe Lys Glu Ser Pro Lys Gln Ile Leu Asp Pro Ala Ala Arg Val Thr
           1070                1075                1080
   GGA ATG AAG AAG TGG CCA AGA ACG CCT AAG GAA GAG GCC CAG TCA CTA      3493
   Gly Met Lys Lys Trp Pro Arg Thr Pro Lys Glu Glu Ala Gln Ser Leu
       1085                1090                1095
   GAA GAC CTG GCT GGC TTC AAA GAG CTC TTC CAG ACA CCA GGT CCC TCT      3541
   Glu Asp Leu Ala Gly Phe Lys Glu Leu Phe Gln Thr Pro Gly Pro Ser
   1100                1105                1110                1115
   GAG GAA TCA ATG ACT GAT GAG AAA ACT ACC AAA ATA GCC TGC AAA TCT      3589
   Glu Glu Ser Met Thr Asp Glu Lys Thr Thr Lys Ile Ala Cys Lys Ser
                   1120                1125                1130
   CCA CCA CCA GAA TCA GTG GAC ACT CCA ACA AGC ACA AAG CAA TGG CCT      3637
   Pro Pro Pro Glu Ser Val Asp Thr Pro Thr Ser Thr Lys Gln Trp Pro
               1135                1140                1145
   AAG AGA AGT CTC AGG AAA GCA GAT GTA GAG GAA GAA TTC TTA GCA CTC      3685
   Lys Arg Ser Leu Arg Lys Ala Asp Val Glu Glu Glu Phe Leu Ala Leu
           1150                1155                1160
   AGG AAA CTA ACA CCA TCA GCA GGG AAA GCC ATG CTT ACG CCC AAA CCA      3733
   Arg Lys Leu Thr Pro Ser Ala Gly Lys Ala Met Leu Thr Pro Lys Pro
       1165                1170                1175
   GCA GGA GGT GAT GAG AAA GAC ATT AAA GCA TTT ATG GGA ACT CCA GTG      3781
   Ala Gly Gly Asp Glu Lys Asp Ile Lys Ala Phe Met Gly Thr Pro Val
   1180                1185                1190                1195
   CAG AAA CTG GAC CTG GCA GGA ACT TTA CCT GGC AGC AAA AGA CAG CTA      3829
   Gln Lys Leu Asp Leu Ala Gly Thr Leu Pro Gly Ser Lys Arg Gln Leu
                   1200                1205                1210
   CAG ACT CCT AAG GAA AAG GCC CAG GCT CTA GAA GAC CTG GCT GGC TTT      3877
   Gln Thr Pro Lys Glu Lys Ala Gln Ala Leu Glu Asp Leu Ala Gly Phe
               1215                1220                1225
   AAA GAG CTC TTC CAG ACT CCT GGT CAC ACC GAG GAA TTA GTG GCT GCT      3925
   Lys Glu Leu Phe Gln Thr Pro Gly His Thr Glu Glu Leu Val Ala Ala
           1230                1235                1240
   GGT AAA ACC ACT AAA ATA CCC TGC GAC TCT CCA CAG TCA GAC CCA GTG      3973
   Gly Lys Thr Thr Lys Ile Pro Cys Asp Ser Pro Gln Ser Asp Pro Val
       1245                1250                1255
   GAC ACC CCA ACA AGC ACA AAG CAA CGA CCC AAG AGA AGT ATC AGG AAA      4021
   Asp Thr Pro Thr Ser Thr Lys Gln Arg Pro Lys Arg Ser Ile Arg Lys
   1260                1265                1270                1275
   GCA GAT GTA GAG GGA GAA CTC TTA GCG TGC AGG AAT CTA ATG CCA TCA      4069
   Ala Asp Val Glu Gly Glu Leu Leu Ala Cys Arg Asn Leu Met Pro Ser
                   1280                1285                1290
   GCA GGC AAA GCC ATG CAC ACG CCT AAA CCA TCA GTA GGT GAA GAG AAA      4117
   Ala Gly Lys Ala Met His Thr Pro Lys Pro Ser Val Gly Glu Glu Lys
               1295                1300                1305
   GAC ATC ATC ATA TTT GTG GGA ACT CCA GTG CAG AAA CTG GAC CTG ACA      4165
   Asp Ile Ile Ile Phe Val Gly Thr Pro Val Gln Lys Leu Asp Leu Thr
           1310                1315                1320
   GAG AAC TTA ACC GGC AGC AAG AGA CGG CCA CAA ACT CCT AAG GAA GAG      4213
   Glu Asn Leu Thr Gly Ser Lys Arg Arg Pro Gln Thr Pro Lys Glu Glu
       1325                1330                1335
   GCC CAG GCT CTG GAA GAC CTG ACT GGC TTT AAA GAG CTC TTC CAG ACC      4261
   Ala Gln Ala Leu Glu Asp Leu Thr Gly Phe Lys Glu Leu Phe Gln Thr
   1340                1345                1350                1355
   CCT GGT CAT ACT GAA GAA GCA GTG GCT GCT GGC AAA ACT ACT AAA ATG      4309
   Pro Gly His Thr Glu Glu Ala Val Ala Ala Gly Lys Thr Thr Lys Met
                   1360                1365                1370
   CCC TGC GAA TCT TCT CCA CCA GAA TCA GCA GAC ACC CCA ACA AGC ACA      4357
   Pro Cys Glu Ser Ser Pro Pro Glu Ser Ala Asp Thr Pro Thr Ser Thr
               1375                1380                1385
   AGA AGG CAG CCC AAG ACA CCT TTG GAG AAA AGG GAC GTA CAG AAG GAG      4405
   Arg Arg Gln Pro Lys Thr Pro Leu Glu Lys Arg Asp Val Gln Lys Glu
           1390                1395                1400
   CTC TCA GCC CTG AAG AAG CTC ACA CAG ACA TCA GGG GAA ACC ACA CAC      4453
   Leu Ser Ala Leu Lys Lys Leu Thr Gln Thr Ser Gly Glu Thr Thr His
       1405                1410                1415
   ACA GAT AAA GTA CCA GGA GGT GAG GAT AAA AGC ATC AAC GCG TTT AGG      4501
   Thr Asp Lys Val Pro Gly Gly Glu Asp Lys Ser Ile Asn Ala Phe Arg
   1420                1425                1430                1435
   GAA ACT GCA AAA CAG AAA CTG GAC CCA GCA GCA AGT GTA ACT GGT AGC      4549
   Glu Thr Ala Lys Gln Lys Leu Asp Pro Ala Ala Ser Val Thr Gly Ser
                   1440                1445                1450
   AAG AGG CAC CCA AAA ACT AAG GAA AAG GCC CAA CCC CTA GAA GAC CTG      4597
   Lys Arg His Pro Lys Thr Lys Glu Lys Ala Gln Pro Leu Glu Asp Leu
               1455                1460                1465
   GCT GGC TGG AAA GAG CTC TTC CAG ACA CCA GTA TGC ACT GAC AAG CCC      4645
   Ala Gly Trp Lys Glu Leu Phe Gln Thr Pro Val Cys Thr Asp Lys Pro
           1470                1475                1480
   ACG ACT CAC GAG AAA ACT ACC AAA ATA GCC TGC AGA TCA CAA CCA GAC      4693
   Thr Thr His Glu Lys Thr Thr Lys Ile Ala Cys Arg Ser Gln Pro Asp
       1485                1490                1495
   CCA GTG GAC ACA CCA ACA AGC TCC AAG CCA CAG TCC AAG AGA AGT CTC      4741
   Pro Val Asp Thr Pro Thr Ser Ser Lys Pro Gln Ser Lys Arg Ser Leu
   1500                1505                1510                1515
   AGG AAA GTG GAC GTA GAA GAA GAA TTC TTC GCA CTC AGG AAA CGA ACA      4789
   Arg Lys Val Asp Val Glu Glu Glu Phe Phe Ala Leu Arg Lys Arg Thr
                   1520                1525                1530
   CCA TCA GCA GGC AAA GCC ATG CAC ACA CCC AAA CCA GCA GTA AGT GGT      4837
   Pro Ser Ala Gly Lys Ala Met His Thr Pro Lys Pro Ala Val Ser Gly
               1535                1540                1545
   GAG AAA AAC ATC TAC GCA TTT ATG GGA ACT CCA GTG CAG AAA CTG GAC      4885
   Glu Lys Asn Ile Tyr Ala Phe Met Gly Thr Pro Val Gln Lys Leu Asp
           1550                1555                1560
   CTG ACA GAG AAC TTA ACT GGC AGC AAG AGA CGG CTA CAA ACT CCT AAG      4933
   Leu Thr Glu Asn Leu Thr Gly Ser Lys Arg Arg Leu Gln Thr Pro Lys
       1565                1570                1575
   GAA AAG GCC CAG GCT CTA GAA GAC CTG GCT GGC TTT AAA GAG CTC TTC      4981
   Glu Lys Ala Gln Ala Leu Glu Asp Leu Ala Gly Phe Lys Glu Leu Phe
   1580                1585                1590                1595
   CAG ACA CGA GGT CAC ACT GAG GAA TCA ATG ACT AAC GAT AAA ACT GCC        5029
   Gln Thr Arg Gly His Thr Glu Glu Ser Met Thr Asn Asp Lys Thr Ala
                   1600                1605                1610
   AAA GTA GCC TGC AAA TCT TCA CAA CCA GAC CTA GAC AAA AAC CCA GCA        5077
   Lys Val Ala Cys Lys Ser Ser Gln Pro Asp Leu Asp Lys Asn Pro Ala
               1615                1620                1625
   AGC TCC AAG CGA CGG CTC AAG ACA TCC CTG GGG AAA GTG GGC GTG AAA        5125
   Ser Ser Lys Arg Arg Leu Lys Thr Ser Leu Gly Lys Val Gly Val Lys
           1630                1635                1640
   GAA GAG CTC CTA GCA GTT GGC AAG CTC ACA CAG ACA TCA GGA GAG ACT        5173
   Glu Glu Leu Leu Ala Val Gly Lys Leu Thr Gln Thr Ser Gly Glu Thr
       1645                1650                1655
   ACA CAC ACA CAC ACA GAG CCA ACA GGA GAT GGT AAG AGC ATG AAA GCA        5221
   Thr His Thr His Thr Glu Pro Thr Gly Asp Gly Lys Ser Met Lys Ala
   1660                1665                1670                1675
   TTT ATG GAG TCT CCA AAG CAG ATC TTA GAC TCA GCA GCA AGT CTA ACT        5269
   Phe Met Glu Ser Pro Lys Gln Ile Leu Asp Ser Ala Ala Ser Leu Thr
                   1680                1685                1690
   GGC AGC AAG AGG CAG CTG AGA ACT CCT AAG GGA AAG TCT GAA GTC CCT        5317
   Gly Ser Lys Arg Gln Leu Arg Thr Pro Lys Gly Lys Ser Glu Val Pro
               1695                1700                1705
   GAA GAC CTG GCC GGC TTC ATC GAG CTC TTC CAG ACA CCA AGT CAC ACT        5365
   Glu Asp Leu Ala Gly Phe Ile Glu Leu Phe Gln Thr Pro Ser His Thr
           1710                1715                1720
   AAG GAA TCA ATG ACT AAT GAA AAA ACT ACC AAA GTA TCC TAC AGA GCT        5413
   Lys Glu Ser Met Thr Asn Glu Lys Thr Thr Lys Val Ser Tyr Arg Ala
       1725                1730                1735
   TCA CAG CCA GAC CTA GTG GAC ACC CCA ACA AGC TCC AAG CCA CAG CCC        5461
   Ser Gln Pro Asp Leu Val Asp Thr Pro Thr Ser Ser Lys Pro Gln Pro
   1740                1745                1750                1755
   AAG AGA AGT CTC AGG AAA GCA GAC ACT GAA GAA GAA TTT TTA GCA TTT        5509
   Lys Arg Ser Leu Arg Lys Ala Asp Thr Glu Glu Glu Phe Leu Ala Phe
                   1760                1765                1770
   AGG AAA CAA ACG CCA TCA GCA GGC AAA GCC ATG CAC ACA CCC AAA CCA        5557
   Arg Lys Gln Thr Pro Ser Ala Gly Lys Ala Met His Thr Pro Lys Pro
               1775                1780                1785
   GCA GTA GGT GAA GAG AAA GAC ATC AAC ACG TTT TTG GGA ACT CCA GTG        5605
   Ala Val Gly Glu Glu Lys Asp Ile Asn Thr Phe Leu Gly Thr Pro Val
           1790                1795                1800
   CAG AAA CTG GAC CAG CCA GGA AAT TTA CCT GGC AGC AAT AGA CGG CTA        5653
   Gln Lys Leu Asp Gln Pro Gly Asn Leu Pro Gly Ser Asn Arg Arg Leu
       1805                1810                1815
   CAA ACT CGT AAG GAA AAG GCC CAG GCT CTA GAA GAA CTG ACT GGC TTC        5701
   Gln Thr Arg Lys Glu Lys Ala Gln Ala Leu Glu Glu Leu Thr Gly Phe
   1820                1825                1830                1835
   AGA GAG CTT TTC CAG ACA CCA TGC ACT GAT AAC CCC ACA GCT GAT GAG        5749
   Arg Glu Leu Phe Gln Thr Pro Cys Thr Asp Asn Pro Thr Ala Asp Glu
                   1840                1845                1850
   AAA ACT ACC AAA AAA ATA CTC TGC AAA TCT CCG CAA TCA GAC CCA GCG      5797
   Lys Thr Thr Lys Lys Ile Leu Cys Lys Ser Pro Gln Ser Asp Pro Ala
               1855                1860                1865
   GAC ACC CCA ACA AAC ACA AAG CAA CGG CCC AAG AGA AGC CTC AAG AAA      5845
   Asp Thr Pro Thr Asn Thr Lys Gln Arg Pro Lys Arg Ser Leu Lys Lys
           1870                1875                1880
   GCA GAC GTA GAG GAA GAA TTT TTA GCA TTC AGG AAA CTA ACA CCA TCA      5893
   Ala Asp Val Glu Glu Glu Phe Leu Ala Phe Arg Lys Leu Thr Pro Ser
       1885                1890                1895
   GCA GGC AAA GCC ATG CAC ACG CCT AAA GCA GCA GTA GGT GAA GAG AAA      5941
   Ala Gly Lys Ala Met His Thr Pro Lys Ala Ala Val Gly Glu Glu Lys
   1900                1905                1910                1915
   GAC ATC AAC ACA TTT GTG GGG ACT CCA GTG GAG AAA CTG GAC CTG CTA      5989
   Asp Ile Asn Thr Phe Val Gly Thr Pro Val Glu Lys Leu Asp Leu Leu
                   1920                1925                1930
   GGA AAT TTA CCT GGC AGC AAG AGA CGG CCA CAA ACT CCT AAA GAA AAG      6037
   Gly Asn Leu Pro Gly Ser Lys Arg Arg Pro Gln Thr Pro Lys Glu Lys
               1935                1940                1945
   GCC AAG GCT CTA GAA GAT CTG GCT GGC TTC AAA GAG CTC TTC CAG ACA      6085
   Ala Lys Ala Leu Glu Asp Leu Ala Gly Phe Lys Glu Leu Phe Gln Thr
           1950                1955                1960
   CCA GGT CAC ACT GAG GAA TCA ATG ACC GAT GAC AAA ATC ACA GAA GTA      6133
   Pro Gly His Thr Glu Glu Ser Met Thr Asp Asp Lys Ile Thr Glu Val
       1965                1970                1975
   TCC TGC AAA TCT CCA CAA CCA GAC CCA GTC AAA ACC CCA ACA AGC TCC      6181
   Ser Cys Lys Ser Pro Gln Pro Asp Pro Val Lys Thr Pro Thr Ser Ser
   1980                1985                1990                1995
   AAG CAA CGA CTC AAG ATA TCC TTG GGG AAA GTA GGT GTG AAA GAA GAG      6229
   Lys Gln Arg Leu Lys Ile Ser Leu Gly Lys Val Gly Val Lys Glu Glu
                   2000                2005                2010
   GTC CTA CCA GTC GGC AAG CTC ACA CAG ACG TCA GGG AAG ACC ACA CAG      6277
   Val Leu Pro Val Gly Lys Leu Thr Gln Thr Ser Gly Lys Thr Thr Gln
               2015                2020                2025
   ACA CAC AGA GAG ACA GCA GGA GAT GGA AAG AGC ATC AAA GCG TTT AAG      6325
   Thr His Arg Glu Thr Ala Gly Asp Gly Lys Ser Ile Lys Ala Phe Lys
           2030                2035                2040
   GAA TCT GCA AAG CAG ATG CTG GAC CCA GCA AAC TAT GGA ACT GGG ATG      6373
   Glu Ser Ala Lys Gln Met Leu Asp Pro Ala Asn Tyr Gly Thr Gly Met
       2045                2050                2055
   GAG AGG TGG CCA AGA ACA CCT AAG GAA GAG GCC CAA TCA CTA GAA GAC      6421
   Glu Arg Trp Pro Arg Thr Pro Lys Glu Glu Ala Gln Ser Leu Glu Asp
   2060                2065                2070                2075
   CTG GCC GGC TTC AAA GAG CTC TTC CAG ACA CCA GAC CAC ACT GAG GAA      6469
   Leu Ala Gly Phe Lys Glu Leu Phe Gln Thr Pro Asp His Thr Glu Glu
                   2080                2085                2090
   TCA ACA ACT GAT GAC AAA ACT ACC AAA ATA GCC TGC AAA TCT CCA CCA      6517
   Ser Thr Thr Asp Asp Lys Thr Thr Lys Ile Ala Cys Lys Ser Pro Pro
               2095                2100                2105
   CCA GAA TCA ATG GAC ACT CCA ACA AGC ACA AGG AGG CGG CCC AAA ACA        6565
   Pro Glu Ser Met Asp Thr Pro Thr Ser Thr Arg Arg Arg Pro Lys Thr
           2110                2115                2120
   CCT TTG GGG AAA AGG GAT ATA GTG GAA GAG CTC TCA GCC CTG AAG CAG        6613
   Pro Leu Gly Lys Arg Asp Ile Val Glu Glu Leu Ser Ala Leu Lys Gln
       2125                2130                2135
   CTC ACA CAG ACC ACA CAC ACA GAC AAA GTA CCA GGA GAT GAG GAT AAA        6661
   Leu Thr Gln Thr Thr His Thr Asp Lys Val Pro Gly Asp Glu Asp Lys
   2140                2145                2150                2155
   GGC ATC AAC GTG TTC AGG GAA ACT GCA AAA CAG AAA CTG GAC CCA GCA        6709
   Gly Ile Asn Val Phe Arg Glu Thr Ala Lys Gln Lys Leu Asp Pro Ala
                   2160                2165                2170
   GCA AGT GTA ACT GGT AGC AAG AGG CAG CCA AGA ACT CCT AAG GGA AAA        6757
   Ala Ser Val Thr Gly Ser Lys Arg Gln Pro Arg Thr Pro Lys Gly Lys
               2175                2180                2185
   GCC CAA CCC CTA GAA GAC TTG GCT GGC TTG AAA GAG CTC TTC CAG ACA        6805
   Ala Gln Pro Leu Glu Asp Leu Ala Gly Leu Lys Glu Leu Phe Gln Thr
           2190                2195                2200
   CCA GTA TGC ACT GAC AAG CCC ACG ACT CAC GAG AAA ACT ACC AAA ATA        6853
   Pro Val Cys Thr Asp Lys Pro Thr Thr His Glu Lys Thr Thr Lys Ile
       2205                2210                2215
   GCC TGC AGA TCT CCA CAA CCA GAC CCA GTG GGT ACC CCA ACA ATC TTC        6901
   Ala Cys Arg Ser Pro Gln Pro Asp Pro Val Gly Thr Pro Thr Ile Phe
   2220                2225                2230                2235
   AAG CCA CAG TCC AAG AGA AGT CTC AGG AAA GCA GAC GTA GAG GAA GAA        6949
   Lys Pro Gln Ser Lys Arg Ser Leu Arg Lys Ala Asp Val Glu Glu Glu
                   2240                2245                2250
   TCC TTA GCA CTC AGG AAA CGA ACA CCA TCA GTA GGG AAA GCT ATG GAC        6997
   Ser Leu Ala Leu Arg Lys Arg Thr Pro Ser Val Gly Lys Ala Met Asp
               2255                2260                2265
   ACA CCC AAA CCA GCA GGA GGT GAT GAG AAA GAC ATG AAA GCA TTT ATG        7045
   Thr Pro Lys Pro Ala Gly Gly Asp Glu Lys Asp Met Lys Ala Phe Met
           2270                2275                2280
   GGA ACT CCA GTG CAG AAA TTG GAC CTG CCA GGA AAT TTA CCT GGC AGC        7093
   Gly Thr Pro Val Gln Lys Leu Asp Leu Pro Gly Asn Leu Pro Gly Ser
       2285                2290                2295
   AAA AGA TGG CCA CAA ACT CCT AAG GAA AAG GCC CAG GCT CTA GAA GAC        7141
   Lys Arg Trp Pro Gln Thr Pro Lys Glu Lys Ala Gln Ala Leu Glu Asp
   2300                2305                2310                2315
   CTG GCT GGC TTC AAA GAG CTC TTC CAG ACA CCA GGC ACT GAC AAG CCC        7189
   Leu Ala Gly Phe Lys Glu Leu Phe Gln Thr Pro Gly Thr Asp Lys Pro
                   2320                2325                2330
   ACG ACT GAT GAG AAA ACT ACC AAA ATA GCC TGC AAA TCT CCA CAA CCA        7237
   Thr Thr Asp Glu Lys Thr Thr Lys Ile Ala Cys Lys Ser Pro Gln Pro
               2335                2340                2345
   GAC CCA GTG GAC ACC CCA GCA AGC ACA AAG CAA CGG CCC AAG AGA AAC        7285
   Asp Pro Val Asp Thr Pro Ala Ser Thr Lys Gln Arg Pro Lys Arg Asn
           2350                2355                2360
   CTC AGG AAA GCA GAC GTA GAG GAA GAA TTT TTA GCA CTC AGG AAA CGA    7333
   Leu Arg Lys Ala Asp Val Glu Glu Glu Phe Leu Ala Leu Arg Lys Arg
       2365                2370                2375
   ACA CCA TCA GCA GGC AAA GCC ATG GAC ACC CCA AAA CCA GCA GTA AGT    7381
   Thr Pro Ser Ala Gly Lys Ala Met Asp Thr Pro Lys Pro Ala Val Ser
   2380                2385                2390                2395
   GAT GAG AAA AAT ATC AAC ACA TTT GTG GAA ACT CCA GTG CAG AAA CTG    7429
   Asp Glu Lys Asn Ile Asn Thr Phe Val Glu Thr Pro Val Gln Lys Leu
                   2400                2405                2410
   GAC CTG CTA GGA AAT TTA CCT GGC AGC AAG AGA CAG CCA CAG ACT CCT    7477
   Asp Leu Leu Gly Asn Leu Pro Gly Ser Lys Arg Gln Pro Gln Thr Pro
               2415                2420                2425
   AAG GAA AAG GCT GAG GCT CTA GAG GAC CTG GTT GGC TTC AAA GAA CTC    7525
   Lys Glu Lys Ala Glu Ala Leu Glu Asp Leu Val Gly Phe Lys Glu Leu
           2430                2435                2440
   TTC CAG ACA CCA GGT CAC ACT GAG GAA TCA ATG ACT GAT GAC AAA ATC    7573
   Phe Gln Thr Pro Gly His Thr Glu Glu Ser Met Thr Asp Asp Lys Ile
       2445                2450                2455
   ACA GAA GTA TCC TGT AAA TCT CCA CAG CCA GAG TCA TTC AAA ACC TCA    7621
   Thr Glu Val Ser Cys Lys Ser Pro Gln Pro Glu Ser Phe Lys Thr Ser
   2460                2465                2470                2475
   AGA AGC TCC AAG CAA AGG CTC AAG ATA CCC CTG GTG AAA GTG GAC ATG    7669
   Arg Ser Ser Lys Gln Arg Leu Lys Ile Pro Leu Val Lys Val Asp Met
                   2480                2485                2490
   AAA GAA GAG CCC CTA GCA GTC AGC AAG CTC ACA CGG ACA TCA GGG GAG    7717
   Lys Glu Glu Pro Leu Ala Val Ser Lys Leu Thr Arg Thr Ser Gly Glu
               2495                2500                2505
   ACT ACG CAA ACA CAC ACA GAG CCA ACA GGA GAT AGT AAG AGC ATC AAA    7765
   Thr Thr Gln Thr His Thr Glu Pro Thr Gly Asp Ser Lys Ser Ile Lys
           2510                2515                2520
   GCG TTT AAG GAG TCT CCA AAG CAG ATC CTG GAC CCA GCA GCA AGT GTA    7813
   Ala Phe Lys Glu Ser Pro Lys Gln Ile Leu Asp Pro Ala Ala Ser Val
       2525                2530                2535
   ACT GGT AGC AGG AGG CAG CTG AGA ACT CGT AAG GAA AAG GCC CGT GCT    7861
   Thr Gly Ser Arg Arg Gln Leu Arg Thr Arg Lys Glu Lys Ala Arg Ala
   2540                2545                2550                2555
   CTA GAA GAC CTG GTT GAC TTC AAA GAG CTC TTC TCA GCA CCA GGT CAC    7909
   Leu Glu Asp Leu Val Asp Phe Lys Glu Leu Phe Ser Ala Pro Gly His
                   2560                2565                2570
   ACT GAA GAG TCA ATG ACT ATT GAC AAA AAC ACA AAA ATT CCC TGC AAA    7957
   Thr Glu Glu Ser Met Thr Ile Asp Lys Asn Thr Lys Ile Pro Cys Lys
               2575                2580                2585
   TCT CCC CCA CCA GAA CTA ACA GAC ACT GCC ACG AGC ACA AAG AGA TGC    8005
   Ser Pro Pro Pro Glu Leu Thr Asp Thr Ala Thr Ser Thr Lys Arg Cys
           2590                2595                2600
   CCC AAG ACA CGT CCC AGG AAA GAA GTA AAA GAG GAG CTC TCA GCA GTT    8053
   Pro Lys Thr Arg Pro Arg Lys Glu Val Lys Glu Glu Leu Ser Ala Val
       2605                2610                2615
   GAG AGG CTC ACG CAA ACA TCA GGG CAA AGC ACA CAC ACA CAC AAA GAA      8101
   Glu Arg Leu Thr Gln Thr Ser Gly Gln Ser Thr His Thr His Lys Glu
   2620                2625                2630                2635
   CCA GCA AGC GGT GAT GAG GGC ATC AAA GTA TTG AAG CAA CGT GCA AAG      8149
   Pro Ala Ser Gly Asp Glu Gly Ile Lys Val Leu Lys Gln Arg Ala Lys
               2640                2645                2650
   AAG AAA CCA AAC CCA GTA GAA GAG GAA CCC AGC AGG AGA AGG CCA AGA      8197
   Lys Lys Pro Asn Pro Val Glu Glu Glu Pro Ser Arg Arg Arg Pro Arg
           2655                2660                2665
   GCA CCT AAG GAA AAG GCC CAA CCC CTG GAA GAC CTG GCC GGC TTC ACA      8245
   Ala Pro Lys Glu Lys Ala Gln Pro Leu Glu Asp Leu Ala Gly Phe Thr
       2670                2675                2680
   GAG CTC TCT GAA ACA TCA GGT CAC ACT CAG GAA TCA CTG ACT GCT GGC      8293
   Glu Leu Ser Glu Thr Ser Gly His Thr Gln Glu Ser Leu Thr Ala Gly
       2685                2690                2695
   AAA GCC ACT AAA ATA CCC TGC GAA TCT CCC CCA CTA GAA GTG GTA GAC      8341
   Lys Ala Thr Lys Ile Pro Cys Glu Ser Pro Pro Leu Glu Val Val Asp
   2700                2705                2710                2715
   ACC ACA GCA AGC ACA AAG AGG CAT CTC AGG ACA CGT GTG CAG AAG GTA      8389
   Thr Thr Ala Ser Thr Lys Arg His Leu Arg Thr Arg Val Gln Lys Val
               2720                2725                2730
   CAA GTA AAA GAA GAG CCT TCA GCA GTC AAG TTC ACA CAA ACA TCA GGG      8437
   Gln Val Lys Glu Glu Pro Ser Ala Val Lys Phe Thr Gln Thr Ser Gly
           2735                2740                2745
   GAA ACC ACG GAT GCA GAC AAA GAA CCA GCA GGT GAA GAT AAA GGC ATC      8485
   Glu Thr Thr Asp Ala Asp Lys Glu Pro Ala Gly Glu Asp Lys Gly Ile
       2750                2755                2760
   AAA GCA TTG AAG GAA TCT GCA AAA CAG ACA CCG GCT CCA GCA GCA AGT      8533
   Lys Ala Leu Lys Glu Ser Ala Lys Gln Thr Pro Ala Pro Ala Ala Ser
       2765                2770                2775
   GTA ACT GGC AGC AGG AGA CGG CCA AGA GCA CCC AGG GAA AGT GCC CAA      8581
   Val Thr Gly Ser Arg Arg Arg Pro Arg Ala Pro Arg Glu Ser Ala Gln
   2780                2785                2790                2795
   GCC ATA GAA GAC CTA GCT GGC TTC AAA GAC CCA GCA GCA GGT CAC ACT      8629
   Ala Ile Glu Asp Leu Ala Gly Phe Lys Asp Pro Ala Ala Gly His Thr
               2800                2805                2810
   GAA GAA TCA ATG ACT GAT GAC AAA ACC ACT AAA ATA CCC TGC AAA TCA      8677
   Glu Glu Ser Met Thr Asp Asp Lys Thr Thr Lys Ile Pro Cys Lys Ser
           2815                2820                2825
   TCA CCA GAA CTA GAA GAC ACC GCA ACA AGC TCA AAG AGA CGG CCC AGG      8725
   Ser Pro Glu Leu Glu Asp Thr Ala Thr Ser Ser Lys Arg Arg Pro Arg
       2830                2835                2840
   ACA CGT GCC CAG AAA GTA GAA GTG AAG GAG GAG CTG TTA GCA GTT GGC      8773
   Thr Arg Ala Gln Lys Val Glu Val Lys Glu Glu Leu Leu Ala Val Gly
       2845                2850                2855
   AAG CTC ACA CAA ACC TCA GGG GAG ACC ACG CAC ACC GAC AAA GAG CCG      8821
   Lys Leu Thr Gln Thr Ser Gly Glu Thr Thr His Thr Asp Lys Glu Pro
   2860                2865                2870                2875
   GTA GGT GAG GGC AAA GGC ACG AAA GCA TTT AAG CAA CCT GCA AAG CGG       8869
   Val Gly Glu Gly Lys Gly Thr Lys Ala Phe Lys Gln Pro Ala Lys Arg
                   2880                     2885                     2890
   AAC GTG GAC GCA GAA GAT GTA ATT GGC AGC AGG AGA CAG CCA AGA GCA       8917
   Asn Val Asp Ala Glu Asp Val Ile Gly Ser Arg Arg Gln Pro Arg Ala
               2895                     2900                     2905
   CCT AAG GAA AAG GCC CAA CCC CTG GAA GAC CTG GCC AGC TTC CAA GAG       8965
   Pro Lys Glu Lys Ala Gln Pro Leu Glu Asp Leu Ala Ser Phe Gln Glu
           2910                     2915                     2920
   CTC TCT CAA ACA CCA GGC CAC ACT GAG GAA CTG GCA AAT GGT GCT GCT       9013
   Leu Ser Gln Thr Pro Gly His Thr Glu Glu Leu Ala Asn Gly Ala Ala
       2925                     2930                     2935
   GAT AGC TTT ACA AGC GCT CCA AAG CAA ACA CCT GAC AGT GGA AAA CCT       9061
   Asp Ser Phe Thr Ser Ala Pro Lys Gln Thr Pro Asp Ser Gly Lys Pro
   2940                     2945                     2950                     2955
   CTA AAA ATA TCC AGA AGA GTT CTT CGG GCC CCT AAA GTA GAA CCC GTG       9109
   Leu Lys Ile Ser Arg Arg Val Leu Arg Ala Pro Lys Val Glu Pro Val
                   2960                     2965                     2970
   GGA GAC GTG GTA AGC ACC AGA GAC CCT GTA AAA TCA CAA AGC AAA AGC       9157
   Gly Asp Val Val Ser Thr Arg Asp Pro Val Lys Ser Gln Ser Lys Ser
               2975                     2980                     2985
   AAC ACT TCC CTG CCC CCA CTG CCC TTC AAG AGG GGA GGT GGC AAA GAT       9205
   Asn Thr Ser Leu Pro Pro Leu Pro Phe Lys Arg Gly Gly Gly Lys Asp
           2990                     2995                     3000
   GGA AGC GTC ACG GGA ACC AAG AGG CTG CGC TGC ATG CCA GCA CCA GAG       9253
   Gly Ser Val Thr Gly Thr Lys Arg Leu Arg Cys Met Pro Ala Pro Glu
       3005                     3010                     3015
   GAA ATT GTG GAG GAG CTG CCA GCC AGC AAG AAG CAG AGG GTT GCT CCC       9301
   Glu Ile Val Glu Glu Leu Pro Ala Ser Lys Lys Gln Arg Val Ala Pro
   3020                     3025                     3030                     3035
   AGG GCA AGA GGC AAA TCA TCC GAA CCC GTG GTC ATC ATG AAG AGA AGT       9349
   Arg Ala Arg Gly Lys Ser Ser Glu Pro Val Val Ile Met Lys Arg Ser
                   3040                     3045                     3050
   TTG AGG ACT TCT GCA AAA AGA ATT GAA CCT GCG GAA GAG CTG AAC AGC       9397
   Leu Arg Thr Ser Ala Lys Arg Ile Glu Pro Ala Glu Glu Leu Asn Ser
               3055                     3060                     3065
   AAC GAC ATG AAA ACC AAC AAA GAG GAA CAC AAA TTA CAA GAC TCG GTC       9445
   Asn Asp Met Lys Thr Asn Lys Glu Glu His Lys Leu Gln Asp Ser Val
           3070                     3075                     3080
   CCT GAA AAT AAG GGA ATA TCC CTG CGC TCC AGA CGC CAA GAT AAG ACT       9493
   Pro Glu Asn Lys Gly Ile Ser Leu Arg Ser Arg Arg Gln Asp Lys Thr
       3085                     3090                     3095
   GAG GCA GAA CAG CAA ATA ACT GAG GTC TTT GTA TTA GCA GAA AGA ATA       9541
   Glu Ala Glu Gln Gln Ile Thr Glu Val Phe Val Leu Ala Glu Arg Ile
   3100                     3105                     3110                     3115
   GAA ATA AAC AGA AAT GAA AAG AAG CCC ATG AAG ACC TCC CCA GAG ATG       9589
   Glu Ile Asn Arg Asn Glu Lys Lys Pro Met Lys Thr Ser Pro Glu Met
                   3120                     3125                     3130
   GAC ATT CAG AAT CCA GAT GAT GGA GCC CGG AAA CCC ATA CCT AGA GAC        9637
   Asp Ile Gln Asn Pro Asp Asp Gly Ala Arg Lys Pro Ile Pro Arg Asp
                   3135                3140                3145
   AAA GTC ACT GAG AAC AAA AGG TGC TTG AGG TCT GCT AGA CAG AAT GAG        9685
   Lys Val Thr Glu Asn Lys Arg Cys Leu Arg Ser Ala Arg Gln Asn Glu
           3150                3155                3160
   AGC TCC CAG CCT AAG GTG GCA GAG GAG AGC GGA GGG CAG AAG AGT GCG        9733
   Ser Ser Gln Pro Lys Val Ala Glu Glu Ser Gly Gly Gln Lys Ser Ala
       3165                3170                3175
   AAG GTT CTC ATG CAG AAT CAG AAA GGG AAA GGA GAA GCA GGA AAT TCA        9781
   Lys Val Leu Met Gln Asn Gln Lys Gly Lys Gly Glu Ala Gly Asn Ser
   3180                3185                3190                3195
   GAC TCC ATG TGC CTG AGA TCA AGA AAG ACA AAA AGC CAG CCT GCA GCA        9829
   Asp Ser Met Cys Leu Arg Ser Arg Lys Thr Lys Ser Gln Pro Ala Ala
                   3200                3205                3210
   AGC ACT TTG GAG AGC AAA TCT GTG CAG AGA GTA ACG CGG AGT GTC AAG        9877
   Ser Thr Leu Glu Ser Lys Ser Val Gln Arg Val Thr Arg Ser Val Lys
                   3215                3220                3225
   AGG TGT GCA GAA AAT CCA AAG AAG GCT GAG GAC AAT GTG TGT GTC AAG        9925
   Arg Cys Ala Glu Asn Pro Lys Lys Ala Glu Asp Asn Val Cys Val Lys
           3230                3235                3240
   AAA ATA ACA ACC AGA AGT CAT AGG GAC AGT GAA GAT ATT TGACAGAAAA         9974
   Lys Ile Thr Thr Arg Ser His Arg Asp Ser Glu Asp Ile
       3245                3250                3255
   ATCGAACTGG GAAAAATATA ATAAAGTTAG TTTTGTGATA AGTTCTAGTG CAGTTTTTGT     10034
   CATAAATTAC AAGTGAATTC TGTAAGTAAG GCTGTCAGTC TGCTTAAGGG AAGAAAACTT     10094
   TGGATTTGCT GGGTCTGAAT CGGCTTCATA AACTCCACTG GGAGCACTGC TGGGCTCCTG     10154
   GACTGAGAAT AGTTGAACAC CGGGGGCTTT GTGAAGGAGT CTGGGCCAAG GTTTGCCCTC     10214
   AGCTTTGCAG AATGAAGCCT TGAGGTCTGT CACCACCCAC AGCCACCCTA CAGCAGCCTT     10274
   AACTGTGACA CTTGCCACAC TGTGTCGTCG TTTGTTTGCC TATGTTCTCC AGGGCACGGT     10334
   GGCAGGAACA ACTATCCTCG TCTGTCCCAA CACTGAGCAG GCACTCGGTA AACACGAATG     10394
   AATGGATAAG CGCACGGATG AATGGAGCTT ACAAGATCTG TCTTTCCAAT GGCCGGGGGC     10454
   ATTTGGTCCC CAAATTAAGG CTATTGGACA TCTGCACAGG ACAGTCCTAT TTTTGATGTC     10514
   CTTTCCTTTC TGAAAATAAA GTTTTGTGCT TTGGAGAATG ACTCGTGAGC ACATCTTTAG     10574
   GGACCAAGAG TGACTTTCTG TAAGGAGTGA CTCGTGGCTT GCCTTGGTCT CTTGGGAATA     10634
   CTTTTCTAAC TAGGGTTGCT CTCACCTGAG ACATTCTCCA CCCGCGGAAT CTCAGGGTCC     10694
   CAGGCTGTGG GCCATCACGA CCTCAAACTG GCTCCTAATC TCCAGCTTTC CTGTCATTGA     10754
   AAGCTTCGGA AGTTTACTGG CTCTGCTCCC GCCTGTTTTC TTTCTGACTC TATCTGGCAG     10814
   CCCGATGCCA CCCAGTACAG GAAGTGACAC CAGTACTCTG TAAAGCATCA TCATCCTTGG     10874
   AGAGACTGAG CACTCAGCAC CTTCAGCCAC GATTTCAGGA TCGCTTCCTT GTGAGCCGCT     10934
   GCCTCCGAAA TCTCCTTTGA AGCCCAGACA TCTTTCTCCA GCTTCAGACT TGTAGATATA   10994
   ACTCGTTCAT CTTCATTTAC TTTCCACTTT GCCCCCTGTC CTCTCTGTGT TCCCCAAATC   11054
   AGAGAATAGC CCGCCATCCC CCAGATCACC TGTCTGGATT CCTCCCCATT CACCCACCTT   11114
   GCCAGGTGCA GGTGAGGATG GTGCACCAGA CAGGGTAGCT GTCCCCCAAA ATGTGCCCTG   11174
   TGCGGGCAGT GCCCTGTCTC CACGTTTGTT TCCCCAGTGT CTGGCGGGGA GCCAGGTGAC   11234
   ATCATAAATA CTTGCTGAAT GAATGCAGAA ATCAGCGGTA CTGACTTGTA CTATATTGGC   11294
   TGCCATGATA GGGTTCTCAC AGCGTCATCC ATGATCGTAA GGGAGAATGA CATTCTGCTT   11354
   GAGGGAGGGA ATAGAAAGGG GCAGGGAGGG GACATCTGAG GGCTTCACAG GGCTGCAAAG   11414
   GGTACAGGGA TTGCACCAGG GCAGAACAGG GGAGGGTGTT CAAGGAAGAG TGGCTCTTAG   11474
   CAGAGGCACT TTGGAAGGTG TGAGGCATAA ATGCTTCCTT CTACGTAGGC CAACCTCAAA   11534
   ACTTTCAGTA GGAATGTTGC TATGATCAAG TTGTTCTAAC ACTTTAGACT TAGTAGTAAT   11594
   TATGAACCTC ACATAGAAAA ATTTCATCCA GCCATATGCC TGTGGAGTGG AATATTCTGT   11654
   TTAGTAGAAA AATCCTTTAG AGTTCAGCTC TAACCAGAAA TCTTGCTGAA GTATGTCAGC   11714
   ACCTTTTCTC ACCCTGGTAA GTACAGTATT TCAAGAGCAC GCTAAGGGTG GTTTTCATTT   11774
   TACAGGGCTG TTGATGATGG GTTAAAAATG TTCATTTAAG GGCTACCCCC GTGTTTAATA   11834
   GATGAACACC ACTTCTACAC AACCCTCCTT GGTACTGGGG GAGGGAGAGA TCTGACAAAT   11894
   ACTGCCCATT CCCCTAGGCT GACTGGATTT GAGAACAAAT ACCCACCCAT TTCCACCATG   11954
   GTATGGTAAC TTCTCTGAGC TTCAGTTTCC AAGTGAATTT CCATGTAATA GGACATTCCC   12014
   ATTAAATACA AGCTGTTTTT ACTTTTTCGC CTCCCAGGGC CTGTGCGATC TGGTCCCCCA   12074
   GCCTCTCTTG GGCTTTCTTA CACTAACTCT GTACCTACCA TCTCCTGCCT CCCTTAGGCA   12134
   GGCACCTCCA ACCACCACAC ACTCCCTGCT GTTTTCCCTG CCTGGAACTT TCCCACCAGC   12194
   CCCACCAAGA TCATTTCATC CAGTCCTGAG CTCAGCTTAA GGGAGGCTTC TTGCCTGTGG   12254
   GTTCCCTCAC CCCCATGCCT GTCCTCCAGG CTGGGGCAGG TTCTTAGTTT GCCTGGAATT   12314
   GTTCTGTACC TCTTTGTAGC ACGTAGTGTT GTGAAACTAA GCCACTAATT GAGTTTCTGG   12374
   CTCCCCTCCT GGGGTTGTAA GTTTTGTTCA TTCATGAGGG CCGACTGTAT TTCCTGGTTA   12434
   CTGTATCCCA GTGACCAGCC ACAGGAGATG TCCAATAAAG TATGTGATGA AATGGTCTT    12493
(2) ANGABEN ZU SEQ ID NO: 2:
   (i) SEQUENZKENNZEICHEN:
      (A) LÄNGE: 3256 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Trp Pro Thr Arg Arg Leu Val Thr Ile Lys Arg Ser Gly Val Asp
   1 5 10 15
   Gly Pro His Phe Pro Leu Ser Leu Ser Thr Cys Leu Phe Gly Arg Gly
   20 25 30
   Ile Glu Cys Asp Ile Arg Ile Gln Leu Pro Val Val Ser Lys Gln His
   35 40 45
   Cys Lys Val Glu Ile His Glu Gln Glu Ala Ile Leu His Asn Phe Ser
   50 55 60
   Ser Thr Asn Pro Thr Gln Val Asn Gly Ser Val Ile Asp Glu Pro Val
   65 70 75 80
   Arg Leu Lys His Gly Asp Val Ile Thr Ile Ile Asp Arg Ser Phe Arg
   85 90 95
   Tyr Glu Asn Glu Ser Leu Gln Asn Gly Arg Lys Ser Thr Glu Phe Pro
   100 105 110
   Arg Lys Ile Arg Glu Gln Glu Pro Ala Arg Arg Val Ser Arg Ser Ser
   115 120 125
   Phe Ser Ser Asp Pro Asp Glu Lys Ala Gln Asp Ser Lys Ala Tyr Ser
   130 135 140
   Lys Ile Thr Glu Gly Lys Val Ser Gly Asn Pro Gln Val His Ile Lys
   145 150 155 160
   Asn Val Lys Glu Asp Ser Thr Ala Asp Asp Ser Lys Asp Ser Val Ala
   165 170 175
   Gln Gly Thr Thr Asn Val His Ser Ser Glu His Ala Gly Arg Asn Gly
   180 185 190
   Arg Asn Ala Ala Asp Pro Ile Ser Gly Asp Phe Lys Glu Ile Ser Ser
   195 200 205
   Val Lys Leu Val Ser Arg Tyr Gly Glu Leu Lys Ser Val Pro Thr Thr
   210 215 220
   Gln Cys Leu Asp Asn Ser Lys Lys Asn Glu Ser Pro Phe Trp Lys Leu
   225 230 235 240
   Tyr Glu Ser Val Lys Lys Glu Leu Asp Val Lys Ser Gln Lys Glu Asn
   245 250 255
   Val Leu Gln Tyr Cys Arg Lys Ser Gly Leu Gln Thr Asp Tyr Ala Thr
   260 265 270
   Glu Lys Glu Ser Ala Asp Gly Leu Gln Gly Glu Thr Gln Leu Leu Val
   275 280 285
   Ser Arg Lys Ser Arg Pro Lys Ser Gly Gly Ser Gly His Ala Val Ala
   290 295 300
   Glu Pro Ala Ser Pro Glu Gln Glu Leu Asp Gln Asn Lys Gly Lys Gly
   305 310 315 320
   Arg Asp Val Glu Ser Val Gln Thr Pro Ser Lys Ala Val Gly Ala Ser
   325 330 335
   Phe Pro Leu Tyr Glu Pro Ala Lys Met Lys Thr Pro Val Gln Tyr Ser
   340 345 350
   Gln Gln Gln Asn Ser Pro Gln Lys His Lys Asn Lys Asp Leu Tyr Thr
   355 360 365
   Thr Gly Arg Arg Glu Ser Val Asn Leu Gly Lys Ser Glu Gly Phe Lys
   370 375 380
   Ala Gly Asp Lys Thr Leu Thr Pro Arg Lys Leu Ser Thr Arg Asn Arg
   385 390 395 400
   Thr Pro Ala Lys Val Glu Asp Ala Ala Asp Ser Ala Thr Lys Pro Glu
   405 410 415
   Asn Leu Ser Ser Lys Thr Arg Gly Ser Ile Pro Thr Asp Val Glu Val
   420 425 430
   Leu Pro Thr Glu Thr Glu Ile His Asn Glu Pro Phe Leu Thr Leu Trp
   435 440 445
   Leu Thr Gln Val Glu Arg Lys Ile Gln Lys Asp Ser Leu Ser Lys Pro
   450 455 460
   Glu Lys Leu Gly Thr Thr Ala Gly Gln Met Cys Ser Gly Leu Pro Gly
   465 470 475 480
   Leu Ser Ser Val Asp Ile Asn Asn Phe Gly Asp Ser Ile Asn Glu Ser
   485 490 495
   Glu Gly Ile Pro Leu Lys Arg Arg Arg Val Ser Phe Gly Gly His Leu
   500 505 510
   Arg Pro Glu Leu Phe Asp Glu Asn Leu Pro Pro Asn Thr Pro Leu Lys
   515 520 525
   Arg Gly Glu Ala Pro Thr Lys Arg Lys Ser Leu Val Met His Thr Pro
   530 535 540
   Pro Val Leu Lys Lys Ile Ile Lys Glu Gln Pro Gln Pro Ser Gly Lys
   545 550 555 560
   Gln Glu Ser Gly Ser Glu Ile His Val Glu Val Lys Ala Gln Ser Leu
   565 570 575
   Val Ile Ser Pro Pro Ala Pro Ser Pro Arg Lys Thr Pro Val Ala Ser
   580 585 590
   Asp Gln Arg Arg Arg Ser Cys Lys Thr Ala Pro Ala Ser Ser Ser Lys
   595 600 605
   Ser Gln Thr Glu Val Pro Lys Arg Gly Gly Glu Arg Val Ala Thr Cys
   610 615 620
   Leu Gln Lys Arg Val Ser Ile Ser Arg Ser Gln His Asp Ile Leu Gln
   625 630 635 640
   Met Ile Cys Ser Lys Arg Arg Ser Gly Ala Ser Glu Ala Asn Leu Ile
   645 650 655
   Val Ala Lys Ser Trp Ala Asp Val Val Lys Leu Gly Ala Lys Gln Thr
   660 665 670
   Gln Thr Lys Val Ile Lys His Gly Pro Gln Arg Ser Met Asn Lys Arg
   675 680 685
   Gln Arg Arg Pro Ala Thr Pro Lys Lys Pro Val Gly Glu Val His Ser
   690 695 700
   Gln Phe Ser Thr Gly His Ala Asn Ser Pro Cys Thr Ile Ile Ile Gly
   705 710 715 720
   Lys Ala His Thr Glu Lys Val His Val Pro Ala Arg Pro Tyr Arg Val
   725 730 735
   Leu Asn Asn Phe Ile Ser Asn Gln Lys Met Asp Phe Lys Glu Asp Leu
   740 745 750
   Ser Gly Ile Ala Glu Met Phe Lys Thr Pro Val Lys Glu Gln Pro Gln
   755 760 765
   Leu Thr Ser Thr Cys His Ile Ala Ile Ser Asn Ser Glu Asn Leu Leu
   770 775 780
   Gly Lys Gln Phe Gln Gly Thr Asp Ser Gly Glu Glu Pro Leu Leu Pro
   785 790 795 800
   Thr Ser Glu Ser Phe Gly Gly Asn Val Phe Phe Ser Ala Gln Asn Ala
   805 810 815
   Ala Lys Gln Pro Ser Asp Lys Cys Ser Ala Ser Pro Pro Leu Arg Arg
   820 825 830
   Gln Cys Ile Arg Glu Asn Gly Asn Val Ala Lys Thr Pro Arg Asn Thr
   835 840 845
   Tyr Lys Met Thr Ser Leu Glu Thr Lys Thr Ser Asp Thr Glu Thr Glu
   850 855 860
   Pro Ser Lys Thr Val Ser Thr Val Asn Arg Ser Gly Arg Ser Thr Glu
   865 870 875 880
   Phe Arg Asn Ile Gln Lys Leu Pro Val Glu Ser Lys Ser Glu Glu Thr
   885 890 895
   Asn Thr Glu Ile Val Glu Cys Ile Leu Lys Arg Gly Gln Lys Ala Thr
   900 905 910
   Leu Leu Gln Gln Arg Arg Glu Gly Glu Met Lys Glu Ile Glu Arg Pro
   915 920 925
   Phe Glu Thr Tyr Lys Glu Asn Ile Glu Leu Lys Glu Asn Asp Glu Lys
   930 935 940
   Met Lys Ala Met Lys Arg Ser Arg Thr Trp Gly Gln Lys Cys Ala Pro
   945 950 955 960
   Met Ser Asp Leu Thr Asp Leu Lys Ser Leu Pro Asp Thr Glu Leu Met
   965 970 975
   Lys Asp Thr Ala Arg Gly Gln Asn Leu Leu Gln Thr Gln Asp His Ala
   980 985 990
   Lys Ala Pro Lys Ser Glu Lys Gly Lys Ile Thr Lys Met Pro Cys Gln
   995 1000 1005
   Ser Leu Gln Pro Glu Pro Ile Asn Thr Pro Thr His Thr Lys Gln Gln
   1010 1015 1020
   Leu Lys Ala Ser Leu Gly Lys Val Gly Val Lys Glu Glu Leu Leu Ala
   1025 1030 1035 1040
   Val Gly Lys Phe Thr Arg Thr Ser Gly Glu Thr Thr His Thr His Arg
   1045 1050 1055
   Glu Pro Ala Gly Asp Gly Lys Ser Ile Arg Thr Phe Lys Glu Ser Pro
   1060 1065 1070
   Lys Gln Ile Leu Asp Pro Ala Ala Arg Val Thr Gly Met Lys Lys Trp
   1075 1080 1085
   Pro Arg Thr Pro Lys Glu Glu Ala Gln Ser Leu Glu Asp Leu Ala Gly
   1090 1095 1100
   Phe Lys Glu Leu Phe Gln Thr Pro Gly Pro Ser Glu Glu Ser Met Thr
   1105 1110 1115 1120
   Asp Glu Lys Thr Thr Lys Ile Ala Cys Lys Ser Pro Pro Pro Glu Ser
   1125 1130 1135
   Val Asp Thr Pro Thr Ser Thr Lys Gln Trp Pro Lys Arg Ser Leu Arg
   1140 1145 1150
   Lys Ala Asp Val Glu Glu Glu Phe Leu Ala Leu Arg Lys Leu Thr Pro
   1155 1160 1165
   Ser Ala Gly Lys Ala Met Leu Thr Pro Lys Pro Ala Gly Gly Asp Glu
   1170 1175 1180
   Lys Asp Ile Lys Ala Phe Met Gly Thr Pro Val Gln Lys Leu Asp Leu
   1185 1190 1195 1200
   Ala Gly Thr Leu Pro Gly Ser Lys Arg Gln Leu Gln Thr Pro Lys Glu
   1205 1210 1215
   Lys Ala Gln Ala Leu Glu Asp Leu Ala Gly Phe Lys Glu Leu Phe Gln
   1220 1225 1230
   Thr Pro Gly His Thr Glu Glu Leu Val Ala Ala Gly Lys Thr Thr Lys
   1235 1240 1245
   Ile Pro Cys Asp Ser Pro Gln Ser Asp Pro Val Asp Thr Pro Thr Ser
   1250 1255 1260
   Thr Lys Gln Arg Pro Lys Arg Ser Ile Arg Lys Ala Asp Val Glu Gly
   1265 1270 1275 1280
   Glu Leu Leu Ala Cys Arg Asn Leu Met Pro Ser Ala Gly Lys Ala Met
   1285 1290 1295
   His Thr Pro Lys Pro Ser Val Gly Glu Glu Lys Asp Ile Ile Ile Phe
   1300 1305 1310
   Val Gly Thr Pro Val Gln Lys Leu Asp Leu Thr Glu Asn Leu Thr Gly
   1315 1320 1325
   Ser Lys Arg Arg Pro Gln Thr Pro Lys Glu Glu Ala Gln Ala Leu Glu
   1330 1335 1340
   Asp Leu Thr Gly Phe Lys Glu Leu Phe Gln Thr Pro Gly His Thr Glu
   1345 1350 1355 1360
   Glu Ala Val Ala Ala Gly Lys Thr Thr Lys Met Pro Cys Glu Ser Ser
   1365 1370 1375
   Pro Pro Glu Ser Ala Asp Thr Pro Thr Ser Thr Arg Arg Gln Pro Lys
   1380 1385 1390
   Thr Pro Leu Glu Lys Arg Asp Val Gln Lys Glu Leu Ser Ala Leu Lys
   1395 1400 1405
   Lys Leu Thr Gln Thr Ser Gly Glu Thr Thr His Thr Asp Lys Val Pro
   1410 1415 1420
   Gly Gly Glu Asp Lys Ser Ile Asn Ala Phe Arg Glu Thr Ala Lys Gln
   1425 1430 1435 1440
   Lys Leu Asp Pro Ala Ala Ser Val Thr Gly Ser Lys Arg His Pro Lys
   1445 1450 1455
   Thr Lys Glu Lys Ala Gln Pro Leu Glu Asp Leu Ala Gly Trp Lys Glu
   1460 1465 1470
   Leu Phe Gln Thr Pro Val Cys Thr Asp Lys Pro Thr Thr His Glu Lys
   1475 1480 1485
   Thr Thr Lys Ile Ala Cys Arg Ser Gln Pro Asp Pro Val Asp Thr Pro
   1490 1495 1500
   Thr Ser Ser Lys Pro Gln Ser Lys Arg Ser Leu Arg Lys Val Asp Val
   1505 1510 1515 1520
   Glu Glu Glu Phe Phe Ala Leu Arg Lys Arg Thr Pro Ser Ala Gly Lys
   1525 1530 1535
   Ala Met His Thr Pro Lys Pro Ala Val Ser Gly Glu Lys Asn Ile Tyr
   1540 1545 1550
   Ala Phe Met Gly Thr Pro Val Gln Lys Leu Asp Leu Thr Glu Asn Leu
   1555 1560 1565
   Thr Gly Ser Lys Arg Arg Leu Gln Thr Pro Lys Glu Lys Ala Gln Ala
   1570 1575 1580
   Leu Glu Asp Leu Ala Gly Phe Lys Glu Leu Phe Gln Thr Arg Gly His
   1585 1590 1595 1600
   Thr Glu Glu Ser Met Thr Asn Asp Lys Thr Ala Lys Val Ala Cys Lys
   1605 1610 1615
   Ser Ser Gln Pro Asp Leu Asp Lys Asn Pro Ala Ser Ser Lys Arg Arg
   1620 1625 1630
   Leu Lys Thr Ser Leu Gly Lys Val Gly Val Lys Glu Glu Leu Leu Ala
   1635 1640 1645
   Val Gly Lys Leu Thr Gln Thr Ser Gly Glu Thr Thr His Thr His Thr
   1650 1655 1660
   Glu Pro Thr Gly Asp Gly Lys Ser Met Lys Ala Phe Met Glu Ser Pro
   1665 1670 1675 1680
   Lys Gln Ile Leu Asp Ser Ala Ala Ser Leu Thr Gly Ser Lys Arg Gln
   1685 1690 1695
   Leu Arg Thr Pro Lys Gly Lys Ser Glu Val Pro Glu Asp Leu Ala Gly
   1700 1705 1710
   Phe Ile Glu Leu Phe Gln Thr Pro Ser His Thr Lys Glu Ser Met Thr
   1715 1720 1725
   Asn Glu Lys Thr Thr Lys Val Ser Tyr Arg Ala Ser Gln Pro Asp Leu
   1730 1735 1740
   Val Asp Thr Pro Thr Ser Ser Lys Pro Gln Pro Lys Arg Ser Leu Arg
   1745 1750 1755 1760
   Lys Ala Asp Thr Glu Glu Glu Phe Leu Ala Phe Arg Lys Gln Thr Pro
   1765 1770 1775
   Ser Ala Gly Lys Ala Met His Thr Pro Lys Pro Ala Val Gly Glu Glu
   1780 1785 1790
   Lys Asp Ile Asn Thr Phe Leu Gly Thr Pro Val Gln Lys Leu Asp Gln
   1795 1800 1805
   Pro Gly Asn Leu Pro Gly Ser Asn Arg Arg Leu Gln Thr Arg Lys Glu
   1810 1815 1820
   Lys Ala Gln Ala Leu Glu Glu Leu Thr Gly Phe Arg Glu Leu Phe Gln
   1825 1830 1835 1840
   Thr Pro Cys Thr Asp Asn Pro Thr Ala Asp Glu Lys Thr Thr Lys Lys
   1845 1850 1855
   Ile Leu Cys Lys Ser Pro Gln Ser Asp Pro Ala Asp Thr Pro Thr Asn
   1860 1865 1870
   Thr Lys Gln Arg Pro Lys Arg Ser Leu Lys Lys Ala Asp Val Glu Glu
   1875 1880 1885
   Glu Phe Leu Ala Phe Arg Lys Leu Thr Pro Ser Ala Gly Lys Ala Met
   1890 1895 1900
   His Thr Pro Lys Ala Ala Val Gly Glu Glu Lys Asp Ile Asn Thr Phe
   1905 1910 1915 1920
   Val Gly Thr Pro Val Glu Lys Leu Asp Leu Leu Gly Asn Leu Pro Gly
   1925 1930 1935
   Ser Lys Arg Arg Pro Gln Thr Pro Lys Glu Lys Ala Lys Ala Leu Glu
   1940 1945 1950
   Asp Leu Ala Gly Phe Lys Glu Leu Phe Gln Thr Pro Gly His Thr Glu
   1955 1960 1965
   Glu Ser Met Thr Asp Asp Lys Ile Thr Glu Val Ser Cys Lys Ser Pro
   1970 1975 1980
   Gln Pro Asp Pro Val Lys Thr Pro Thr Ser Ser Lys Gln Arg Leu Lys
   1985 1990 1995 2000
   Ile Ser Leu Gly Lys Val Gly Val Lys Glu Glu Val Leu Pro Val Gly
   2005 2010 2015
   Lys Leu Thr Gln Thr Ser Gly Lys Thr Thr Gln Thr His Arg Glu Thr
   2020 2025 2030
   Ala Gly Asp Gly Lys Ser Ile Lys Ala Phe Lys Glu Ser Ala Lys Gln
   2035 2040 2045
   Met Leu Asp Pro Ala Asn Tyr Gly Thr Gly Met Glu Arg Trp Pro Arg
   2050 2055 2060
   Thr Pro Lys Glu Glu Ala Gln Ser Leu Glu Asp Leu Ala Gly Phe Lys
   2065                2070                2075                2080
   Glu Leu Phe Gln Thr Pro Asp His Thr Glu Glu Ser Thr Thr Asp Asp
                   2085                2090                2095
   Lys Thr Thr Lys Ile Ala Cys Lys Ser Pro Pro Pro Glu Ser Met Asp
               2100                2105                2110
   Thr Pro Thr Ser Thr Arg Arg Arg Pro Lys Thr Pro Leu Gly Lys Arg
           2115                2120                2125
   Asp Ile Val Glu Glu Leu Ser Ala Leu Lys Gln Leu Thr Gln Thr Thr
       2130                2135                2140
   His Thr Asp Lys Val Pro Gly Asp Glu Asp Lys Gly Ile Asn Val Phe
   2145                2150                2155                2160
   Arg Glu Thr Ala Lys Gln Lys Leu Asp Pro Ala Ala Ser Val Thr Gly
                   2165                2170                2175
   Ser Lys Arg Gln Pro Arg Thr Pro Lys Gly Lys Ala Gln Pro Leu Glu
               2180                2185                2190
   Asp Leu Ala Gly Leu Lys Glu Leu Phe Gln Thr Pro Val Cys Thr Asp
           2195                2200                2205
   Lys Pro Thr Thr His Glu Lys Thr Thr Lys Ile Ala Cys Arg Ser Pro
       2210                2215                2220
   Gln Pro Asp Pro Val Gly Thr Pro Thr Ile Phe Lys Pro Gln Ser Lys
   2225                2230                2235                2240
   Arg Ser Leu Arg Lys Ala Asp Val Glu Glu Glu Ser Leu Ala Leu Arg
                   2245                2250                2255
   Lys Arg Thr Pro Ser Val Gly Lys Ala Met Asp Thr Pro Lys Pro Ala
               2260                2265                2270
   Gly Gly Asp Glu Lys Asp Met Lys Ala Phe Met Gly Thr Pro Val Gln
           2275                2280                2285
   Lys Leu Asp Leu Pro Gly Asn Leu Pro Gly Ser Lys Arg Trp Pro Gln
       2290                2295                2300
   Thr Pro Lys Glu Lys Ala Gln Ala Leu Glu Asp Leu Ala Gly Phe Lys
   2305                2310                2315                2320
   Glu Leu Phe Gln Thr Pro Gly Thr Asp Lys Pro Thr Thr Asp Glu Lys
                   2325                2330                2335
   Thr Thr Lys Ile Ala Cys Lys Ser Pro Gln Pro Asp Pro Val Asp Thr
               2340                2345                2350
   Pro Ala Ser Thr Lys Gln Arg Pro Lys Arg Asn Leu Arg Lys Ala Asp
           2355                2360                2365
   Val Glu Glu Glu Phe Leu Ala Leu Arg Lys Arg Thr Pro Ser Ala Gly
       2370                2375                2380
   Lys Ala Met Asp Thr Pro Lys Pro Ala Val Ser Asp Glu Lys Asn Ile
   2385                2390                2395                2400
   Asn Thr Phe Val Glu Thr Pro Val Gln Lys Leu Asp Leu Leu Gly Asn
                   2405                2410                2415
   Leu Pro Gly Ser Lys Arg Gln Pro Gln Thr Pro Lys Glu Lys Ala Glu
   2420 2425 2430
   Ala Leu Glu Asp Leu Val Gly Phe Lys Glu Leu Phe Gln Thr Pro Gly
   2435 2440 2445
   His Thr Glu Glu Ser Met Thr Asp Asp Lys Ile Thr Glu Val Ser Cys
   2450 2455 2460
   Lys Ser Pro Gln Pro Glu Ser Phe Lys Thr Ser Arg Ser Ser Lys Gln
   2465 2470 2475 2480
   Arg Leu Lys Ile Pro Leu Val Lys Val Asp Met Lys Glu Glu Pro Leu
   2485 2490 2495
   Ala Val Ser Lys Leu Thr Arg Thr Ser Gly Glu Thr Thr Gln Thr His
   2500 2505 2510
   Thr Glu Pro Thr Gly Asp Ser Lys Ser Ile Lys Ala Phe Lys Glu Ser
   2515 2520 2525
   Pro Lys Gln Ile Leu Asp Pro Ala Ala Ser Val Thr Gly Ser Arg Arg
   2530 2535 2540
   Gln Leu Arg Thr Arg Lys Glu Lys Ala Arg Ala Leu Glu Asp Leu Val
   2545 2550 2555 2560
   Asp Phe Lys Glu Leu Phe Ser Ala Pro Gly His Thr Glu Glu Ser Met
   2565 2570 2575
   Thr Ile Asp Lys Asn Thr Lys Ile Pro Cys Lys Ser Pro Pro Pro Glu
   2580 2585 2590
   Leu Thr Asp Thr Ala Thr Ser Thr Lys Arg Cys Pro Lys Thr Arg Pro
   2595 2600 2605
   Arg Lys Glu Val Lys Glu Glu Leu Ser Ala Val Glu Arg Leu Thr Gln
   2610 2615 2620
   Thr Ser Gly Gln Ser Thr His Thr His Lys Glu Pro Ala Ser Gly Asp
   2625 2630 2635 2640
   Glu Gly Ile Lys Val Leu Lys Gln Arg Ala Lys Lys Lys Pro Asn Pro
   2645 2650 2655
   Val Glu Glu Glu Pro Ser Arg Arg Arg Pro Arg Ala Pro Lys Glu Lys
   2660 2665 2670
   Ala Gln Pro Leu Glu Asp Leu Ala Gly Phe Thr Glu Leu Ser Glu Thr
   2675 2680 2685
   Ser Gly His Thr Gln Glu Ser Leu Thr Ala Gly Lys Ala Thr Lys Ile
   2690 2695 2700
   Pro Cys Glu Ser Pro Pro Leu Glu Val Val Asp Thr Thr Ala Ser Thr
   2705 2710 2715 2720
   Lys Arg His Leu Arg Thr Arg Val Gln Lys Val Gln Val Lys Glu Glu
   2725 2730 2735
   Pro Ser Ala Val Lys Phe Thr Gln Thr Ser Gly Glu Thr Thr Asp Ala
   2740 2745 2750
   Asp Lys Glu Pro Ala Gly Glu Asp Lys Gly Ile Lys Ala Leu Lys Glu
           2755                2760                2765
   Ser Ala Lys Gln Thr Pro Ala Pro Ala Ala Ser Val Thr Gly Ser Arg
       2770                2775                2780
   Arg Arg Pro Arg Ala Pro Arg Glu Ser Ala Gln Ala Ile Glu Asp Leu
   2785                2790                2795                2800
   Ala Gly Phe Lys Asp Pro Ala Ala Gly His Thr Glu Glu Ser Met Thr
                   2805                2810                2815
   Asp Asp Lys Thr Thr Lys Ile Pro Cys Lys Ser Ser Pro Glu Leu Glu
               2820                2825                2830
   Asp Thr Ala Thr Ser Ser Lys Arg Arg Pro Arg Thr Arg Ala Gln Lys
           2835                2840                2845
   Val Glu Val Lys Glu Glu Leu Leu Ala Val Gly Lys Leu Thr Gln Thr
       2850                2855                2860
   Ser Gly Glu Thr Thr His Thr Asp Lys Glu Pro Val Gly Glu Gly Lys
   2865                2870                2875                2880
   Gly Thr Lys Ala Phe Lys Gln Pro Ala Lys Arg Asn Val Asp Ala Glu
                   2885                2890                2895
   Asp Val Ile Gly Ser Arg Arg Gln Pro Arg Ala Pro Lys Glu Lys Ala
               2900                2905                2910
   Gln Pro Leu Glu Asp Leu Ala Ser Phe Gln Glu Leu Ser Gln Thr Pro
           2915                2920                2925
   Gly His Thr Glu Glu Leu Ala Asn Gly Ala Ala Asp Ser Phe Thr Ser
       2930                2935                2940
   Ala Pro Lys Gln Thr Pro Asp Ser Gly Lys Pro Leu Lys Ile Ser Arg
   2945                2950                2955                2960
   Arg Val Leu Arg Ala Pro Lys Val Glu Pro Val Gly Asp Val Val Ser
                   2965                2970                2975
   Thr Arg Asp Pro Val Lys Ser Gln Ser Lys Ser Asn Thr Ser Leu Pro
               2980                2985                2990
   Pro Leu Pro Phe Lys Arg Gly Gly Gly Lys Asp Gly Ser Val Thr Gly
           2995                3000                3005
   Thr Lys Arg Leu Arg Cys Met Pro Ala Pro Glu Glu Ile Val Glu Glu
       3010                3015                3020
   Leu Pro Ala Ser Lys Lys Gln Arg Val Ala Pro Arg Ala Arg Gly Lys
   3025                3030                3035                3040
   Ser Ser Glu Pro Val Val Ile Met Lys Arg Ser Leu Arg Thr Ser Ala
                   3045                3050                3055
   Lys Arg Ile Glu Pro Ala Glu Glu Leu Asn Ser Asn Asp Met Lys Thr
               3060                3065                3070
   Asn Lys Glu Glu His Lys Leu Gln Asp Ser Val Pro Glu Asn Lys Gly
           3075                3080                3085
   Ile Ser Leu Arg Ser Arg Arg Gln Asp Lys Thr Glu Ala Glu Gln Gln
       3090                3095                3100
   Ile Thr Glu Val Phe Val Leu Ala Glu Arg Ile Glu Ile Asn Arg Asn
   3105                3110                3115                3120
   Glu Lys Lys Pro Met Lys Thr Ser Pro Glu Met Asp Ile Gln Asn Pro
                   3125                3130                3135
   Asp Asp Gly Ala Arg Lys Pro Ile Pro Arg Asp Lys Val Thr Glu Asn
               3140                3145                3150
   Lys Arg Cys Leu Arg Ser Ala Arg Gln Asn Glu Ser Ser Gln Pro Lys
           3155                3160                3165
   Val Ala Glu Glu Ser Gly Gly Gln Lys Ser Ala Lys Val Leu Met Gln
       3170                3175                3180
   Asn Gln Lys Gly Lys Gly Glu Ala Gly Asn Ser Asp Ser Met Cys Leu
   3185                3190                3195                3200
   Arg Ser Arg Lys Thr Lys Ser Gln Pro Ala Ala Ser Thr Leu Glu Ser
                   3205                3210                3215
   Lys Ser Val Gln Arg Val Thr Arg Ser Val Lys Arg Cys Ala Glu Asn
               3220                3225                3230
   Pro Lys Lys Ala Glu Asp Asn Val Cys Val Lys Lys Ile Thr Thr Arg
           3235                3240                3245
   Ser His Arg Asp Ser Glu Asp Ile
       3250                3255
(2) ANGABEN ZU SEQ ID NO: 3:
   (i) SEQUENZKENNZEICHEN:
      (A) LÄNGE: 23 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "synthetisches Oligonukleotid"
   (xi) SEQUENZBESCHREIBUNG: SEQ ID NO: 3:
   ACCAGGCGTC TCGTGGGCCA CAT                                               23